(19) Europäisches Patentamt

European Patent Office

Office européen des brevets

(11) **EP 1 649 879 A2**

(12) **EUROPEAN PATENT APPLICATION**

(43) Date of publication:
26.04.2006 Bulletin 2006/17

(51) Int Cl.:
*A61L 27/38* (2006.01)

(21) Application number: 05023619.9

(22) Date of filing: 27.06.2003

(84) Designated Contracting States:
AT BE BG CH CY CZ DE DK EE ES FI FR GB GR
HU IE IT LI LU MC NL PT RO SE SI SK TR

(30) Priority: 28.06.2002 JP 2002191527

(62) Document number(s) of the earlier application(s) in
accordance with Art. 76 EPC:
03736304.1 / 1 517 715

(71) Applicants:
• **Cardio, Inc.**
Osaka-shi,
Osaka 530-0043 (JP)
• **National Institute of Advanced Industrial Science
and Technology**
Tokyo 100-8921 (JP)

(72) Inventors:
• **Sawa, Yoshiki**
Osaka University
2-2, Yamadaoka Suita-shi
Osaka 565-0871 (JP)
• **Taketani, Satoshi**
Osaka-shi
Osaka 530-0043 (JP)
• **Iwai, Shigemitsu**
Osaka University
2-2, Yamadaoka Suita-shi
Osaka 565-0871 (JP)

• **Matsuda, Hikaru**
Osaka University
2-2, Yamadaoka Suita-shi
Osaka 565-0871 (JP)
• **Hara, Masayuki**
National Institute
Amagasaki-shi
Hyogo 661-0974 (JP)
• **Uchimura, Eiichiro**
National Institute
Amagasaki-shi
Hyogo 661-0974 (JP)
• **Miyake, Jun**
National Institute
Amagasaki-shi
Hyogo 661-0974 (JP)

(74) Representative: **Williams, Aylsa**
D Young & Co
120 Holborn
London EC1N 2DY (GB)

Remarks:
This application was filed on 28-10-2005 as a
divisional application to the application mentioned
under INID code 62.

(54) **Decellularized tissue**

(57) An objective of the present invention is to overcome a problem that there is an inverse relationship between the decellularization rate and the strength of tissue. This problem was solved by immersing tissue in a solution containing a non-micellar amphipathic molecule (e.g., a 1,2-epoxide polymer). Thus, the present invention provides decellularized tissue, in which the cell survival rate of the tissue is less than a level at which calcification or an immune reaction is elicited in an organism and the tissue damage rate of the tissue is suppressed to a level which permits clinical applications. Tissue prepared by the above-described treatment preferably retains a certain level of tissue strength. Further, the tissue of the present invention has an effect of performing cell replacement.

**Description**

TECHNICAL FIELD

The present invention relates to a method and system for decellularizing tissue, tissue prepared by the decellularization method, and a medicament and therapeutic method utilizing a tissue graft or the like.

BACKGROUND ART

Implantation of organs (e.g., heart, blood vessel, etc.) derived from exogenous tissue is mainly hindered by immunological rejections. Changes occurring in allografts and xenografts were first described 90 years or more ago (Carrel A., 1907, J. Exp. Med. 9:226-8; Carrel A., 1912., J. Exp. Med. 9:389-92; GuthrieC.C., 1908, J. Am. Med. Assoc; Calne R. Y., 1970, Transplant Proc. 2:550; and Auchincloss 1988, Transplantation 46:1). Rejection to artery grafts pathologically leads either to enlargement (up to rupture) or obstruction of the grafts. The former is caused by decomposition of extracellular matrices, while the latter is caused by proliferation of cells in a blood vessel (Uretsky B. F., Mulari S., Reddy S., et al., 1987, Circulation 76:827-34).

Conventionally, two strategies have been used to alleviate rejection of these substances. One of the two strategies is to reduce the immune reaction of hosts (Schmitz-Rixen T., Megerman J., Colvin R. B., Williams A. M., Abbot W., 1988, J. Vasc. Surg. 7:82-92: and Plissonnier D., et al., 1993, Arteriosclerosis Thromb, 13:112-9). The other is to reduce the antigenicity of allografts or xenografts mainly by cross-linking (Rosenberg N., et al., 1956, Surg. Forum 6: 242-6; and Dumont C., Pissonnier D., Michel J. B., 1993, J. Surg. Res. 54:61-69). The cross-linking of extracellular matrices reduces the antigenicityof grafts, but changes bioengineering functions (Cosgrove D. M., Lytle B. W., Golding C. C., et al., 1983, J. Thorac. Cardiovasc. Surgery 64:172-176; and Broom N., Christie G. W., 1982, In: Cohn L. H., Gallucci V., editors. Cardiac bioprostheses: Proceedings of the Second International Symposium. New York: York Medical Books Pages 476-491), so that the grafts become susceptible to mineralization (Schoen F. J., Levy R. J., Piehler H. R., 1992, Cardiovasc. Pathology 1992; 1:29-52).

Cells in extracellular matrices have Class I and II histocompatibility antigens capable of eliciting rejection reactions. Also, the cells have glycoproteins recognized by the immune system of hosts, which elicit rejection reactions. Therefore, if these substances are eliminated from extracellular matrices, rejection reactions can be prevented. However, complete elimination of all antigens is considerably difficult to perform and verify. Malone et al. (Malone J. M., Brendel K., Duhamel R. C., Reinert R. L., 1984, J. Vasc. Surg. 1:181-91) and Lalka et al. (Lalka S. G., Oelker L. M., and Malone J. M., et al., 1989, Ann Vase. Surg., 3:108-17) reported that although immune reactions were stimulated in matrices to which "cell-free" artery allografts (graft to the same species animal) were implanted, proliferation in blood vessels and acceptance of endothelial cells were also observed. Most recently, O'Brian et al. reported that decellularized porcine tissue can be applied to implantation of cardiac blood vessels and that no hyperacute rejection was elicited when implanted to sheep (O'Brien M. F., et al., 1999 (October), Seminars in Thorac. and Cardiovasc. Surg.; 111(4), Suppl 1: 194-200).

Cardiovascular disease, including coronary artery and peripheral vascular disease, is treated by surgical replacement. The number of cardiovascular disease cases is recently increasing all over the world. In the case of small-diameter blood vessels, it is difficult to apply replacement therapy. In the case of small diameter blood vessels, bypass operations are applied, where an autologous venous or arterial graft is used (Canver C. C., 1995, Chest 1995:108 1150-1155; BarnerH. B., 1998, Ann. Thorac. Surg., 66 (Suppl 5) S2-5, discussion S25-28; and Bhan A., Gupta V., Choudhary S. K., et al., 1999, Ann Thorac. Surg. , 1999:67 1631-1636). Although venous and arterial grafts currently yield the best results, disadvantages include the need for complicated operations and no suitable blood vessels available in patients with certain diseases. As a result, there is a demand for a vascular prosthesis which is suited to the small-diameter blood vessels. In order to reduce the use of auto- or allo-grafts, efforts have been made to develop artificial materials. However, no artificial material is suitable for construction of small-diameter arteries (<6 mm) required for extremity and coronary artery bypass grafting operations. On the other hand, in the case of cardiac blood vessels, the feasibility of native tissue grafts as biomaterials in clinical applications have been investigated. The use of xenograft and allograft tissues typically requires chemical or physical treatment (e.g., glutaraldehyde fixation). Cross-linking techniques have been investigated and found to be an ideal procedure for stabilizing the collagen-based structure of tissue (Hilbert S. L., Ferrans V. J., Jone M., 1988, Med Prog. Technol. 89:14, 115-163).

However, implantation has the problem of calcification when viewed in the long term. This detrimental side effect of calcification in glutaraldehyde treatment is the main cause of failure of bioprosthetic heart valves (Rao K.P., Shanthi C., 1999, Biomaterials Appl., 13:238-268; and Grabenwoger M., Sider J., Fitzal F., et al., 1996, Ann. Thorac. Surg., 62:772-777). As an alternative approach to native tissue graft, an attempt has been made to produce an acellular tissue matrix by specifically removing cellular components which were believed to promote calcification and elicit an immune response. The decellularization technique includes chemical, enzymatic, and mechanical means for removing cellular components. This treatment leaves a material composed essentially of extracellular matrix components. The resultant decellularized tissue retains native mechanical properties and promotes regeneration. Regeneration is caused by neovascularization and recellularization by hosts. Surfactant treatment, which is a typical cell extraction method, has been carried out as a means for creating completely decellularized tissue for use as a biomaterial graft. This is because cellular components, lipids and residual surfactant remaining within treated tissue may promote undesired effects, such as calcification (Valente M., Bortolotti U., Thiene G., 1985, Am. J. Pathol. 119, 12-21; Maranto A. R., Shoen F. J., 1988, ASAIO Trans. 34, 827-830; Courtman D. W., Pereira C. A., Kashef V., McComb D., Lee J. M., WilsonG. L., 1994, J. Biomed. Mater. Res. 28:655-666; and Levy R. J., Schoen F. J., Anderson H. C., et al., 1991, Biomaterials 12:707-714). Removal of lipids from bovine pericardium treated by either chloroform/methanol or sodium dodecyl sulfate (SDS) decreased calcification of tissue in a rat model (Jorge-Herrero E., Fernandez P., Gutierrez M. P., Castillo-Olivares J.L., 1991, Biomaterials 12:683-689). Recently, Sunjay et al. demonstrated that decellularized blood vessel grafts were coated with endothelial progenitor cells (EPCs). This is because these grafts have well preserved extracellular matrices and mechanical properties similar to those of native blood vessels including arteries (Sunjay Kaushal, Gilad E. Amiel, Kristine J., Guleserian, et al., 2001, Nature Medicine Vol. 9, 1035-1040). Sunjay et al. isolated endothelial progenitor cells (EPCs) from peripheral blood and disseminated EPCs in decellularized bovine ileac blood vessels. The EPC-disseminated decellularized graft developed new blood vessels *in vivo* by day 130. The new blood vessels underwent NO-mediated vascular relaxation.

(Problems to be Solved by the Invention)

Decellularized tissue prepared by conventional techniques has a poor decellularization rate. If the decellularization rate is increased, the mechanical strength is lost so that the tissue cannot be satisfactorily used in medical applications. Moreover, the supply of decellularized tissue prepared by conventional techniques is limited, and the strength of the products is low. Therefore, the tissue is unfortunately limited to the right heart arterial system.

Many researchers recognize a number of utilities of decellularized grafts, including physical and biological characteristics. However, it is considered that a further improvement is required for currently available decellularized grafts because of disadvantages, such as calcification and immune response (Christine E. Schmidt, Jennie M. Baier, 2000, Biomaterials 21:2215-2231). Also, residual surfactant in grafts prepared by the above-described processing techniques is absorbed into a decellularized tissue matrix, leading to a large problem in clinical applications.

DISCLOSURE OF THE INVENTION

The present inventors herein disclose completely new decellularization technology. The present invention provides a novel method for removing cell components without a toxic surfactant which raises a problem in clinical applications. The present inventors have diligently studied and successfully developed a method for decellularization without a surfactant.

The above-described problems have been solved by immersing provided tissue in a solution containing amphipathic molecules (e.g., 1,2-epoxide polymer) which are not in the micelle form. Therefore, the present invention relates to a novel method for decellularizing porcine aortic valve tissue to use it *ex vivo* or *in vivo* as a scaffold for constructing cardiovascular/vascular bioprostheses; tissue; tissue grafts; and methods using them. The decellularization may be used for various purposes with or without cells (self or non-self cells).

The present inventors established a two-step strategy for extracting cell components from blood vessel tissue without a conventional surfactant. In step 1, cytosol and cell membrane are extracted using amphipathic molecules (e.g., 1,2-epoxide polymers such as polyethylene glycol (PEG)) which are not in the micelle form. 1,2-epoxide polymers such as polyethylene glycol (PEG) have the effect of unstabilizing the cell membrane which separates the inside from the outside of a cell, so that most cell membrane components and cytosol (water soluble components) are removed in step 1. In step 2, nucleic acid components are enzymatically decomposed and deposited from the extracts.

The present invention is fundamentally different from conventional techniques utilizing a surfactant in that a solution substantially free from micellar molecules and containing amphipathic molecules which are not in the micelle form is utilized. In conventional techniques, surfactants capable of forming micelles are strongly absorbed to a two-phase interface, so that the free energy of the interface is significantly reduced, thereby removing substances, such as proteins, lipids, and the like. Such a removal technique is comparable to the conception that substances are solubilized and washed out. Thus, conventional techniques have the disadvantageous effects of surfactants, such as poor strength of tissue, the inadequate removal of cell components, and the like.

The above-described problems could be overcome by using amphipathic molecules which are not in the micelle form. This effect was achieved by an absolutely novel conception that non-micellar molecules are used to perform decellularization in a mechanism similar to extraction of cell components. Therefore, any amphipathic molecule that is not in the micelle form can be used in the present invention.

The thus-obtained decellularized tissue has minimum damage to extracellular matrices. The decellularized tissue can be used as a vascular prosthesis on a permanent basis. Further, it was confirmed that after implantation, host-derived cells infiltrate and replace decellularized tissue and grafts prepared with the technique of the present invention. Such an event never occurred in tissue grafts conventionally prepared. This finding per se can be said to indicate an unexpected, excellent effect of the present invention. Furthermore, such a phenomenon is observed either when the decellularized tissue of the present invention is used along with cells or when the decellularized tissue of the present invention is used without a cell. Thus, the utility of the decellularized tissue of the present invention can be said to be extensive.

It was revealed that after the decellularized tissue and tissue graft of the present invention are implanted to a host, host-derived cells replace the tissue and tissue graft. Such cell replacement was absolutely not observed in conventional tissue grafts. The decellularized tissue and tissue graft of the present invention can be said to be used on a permanent basis. These effects are unexpected and cannot be achieved by conventional techniques. The provision of such decellularized tissue and tissue graft can be said to open up significant advances in implantation medicine. The meaning of the present invention is almost beyond description.

Therefore, the present invention provides the following.

According to an aspect of the present invention, decellularized tissue is provided, in which A) a cell survival rate of the tissue is less than a level at which an immune reaction is elicited in an organism; and B) the tissue is not damaged to such an extent that hinders the tissue from exhibiting a function which was possessed by the tissue when the tissue was not decellularized.

In one embodiment of this invention, the the cell survival rate of the tissue is 30% or less.

In one embodiment of this invention, the tissue damage rate of the tissue is 30% or less.

In one embodiment of this invention, the tissue has a tissue strength which permits a clinical application.

In one embodiment of this invention, the tissue has a tissue strength which is 80% or more of a tissue strength which was possessed by the tissue when the tissue was not decellularized.

In one embodiment of this invention, the tissue has a tissue strength having a $\beta$ value which is 80% or more of a $\beta$ value which was possessed by the tissue when the tissue was not decellularized.

In one embodiment of this invention, the tissue has a tissue strength having a $\beta$ value of 20 or more.

In one embodiment of this invention, the tissue is lumenal tissue.

In one embodiment of this invention, the tissue is tissue selectedfromblood vessels,blood vessel-like tissue, cardiac valves, pericardia, dura mater, corneas, and bones.

In one embodiment of this invention, a state of the tissue, in which the tissue is not damaged to such an extent that hinders the tissue from exhibiting a function which was possessed by the tissue when the tissue was not decellularized, includes that an extracellular matrix of the tissue is not substantially degenerated.

In one embodiment of this invention, a survival rate of the extracellular matrix is at least about 50%.

In one embodiment of this invention, the tissue is derived from a mammal.

In one embodiment of this invention, the tissue is derived from a human.

In one embodiment of this invention, the tissue is derived from a swine.

According to another aspect of the present invention, a tissue graft is provided, which comprises A) decellularized tissue, wherein a cell survival rate of the tissue is less than a level at which an immune reaction is elicited in an organism; the tissue is not damaged to such an extent that hinders the tissue from exhibiting a function which was possessed by the tissue when the tissue was not decellularized; and the tissue has a desired structure.

In one embodiment of this invention, the tissue graft comprises a cell.

In one embodiment of this invention, the cell is a recipient-derived cell.

In one embodiment of this invention, the cell is derived from an organism of the same species as the tissue.

In one embodiment of this invention, the tissue graft comprises no cell.

In one embodiment of this invention, the decellularized tissue is tissue of an organ selected from the group consisting of blood vessels, blood vessel-like tissue, cardiac valves, pericardia, dura mater, corneas, and bones.

In one embodiment of this invention, the tissue is derived from a mammal.

In one embodiment of this invention, the tissue is derived from a human.

In one embodiment of this invention, the cell is selected from the group consisting of vascular endothelial cells, smooth muscle cells, fibroblast, blood cells, and precursor cells and somatic stem cells capable of differentiating thereto.

According to another aspect of the present invention, a membrane-like tissue graft is provided, which comprises A) decellularized tissue, wherein a cell survival rate of the tissue is less than a level at which an immune reaction is elicited in an organism; the tissue is not damaged to such an extent that hinders the tissue from exhibiting a function which was possessed by the tissue when the tissue was not decellularized; and the tissue has a desired structure.

In one embodiment of this invention, the tissue graft comprises a cell.

In one embodiment of this invention, the cell is a recipient-derived cell.

In one embodiment of this invention, the cell is derived from an organism of the same species as the tissue.

In one embodiment of this invention, the tissue graft comprises no cell.

According to another aspect of the present invention, a method of producing decellularized tissue is provided, which comprises the steps of: 1) providing tissue; and 2) immersing the tissue in a solution containing a non-micellar amphipathic molecule.

In one embodiment of this invention, the method further comprises 3) washing the tissue.

In one embodiment of this invention, the washing step is performed with PBS.

In one embodiment of this invention, the amphipathic molecule is a 1,2-epoxide polymer.

In one embodiment of this invention, the amphipathic molecule is polyethylene glycol (PEG).

In one embodiment of this invention, an average molecular weight of the PEG is between 200 to 6000.

In one embodiment of this invention, an average molecular weight of the PEG is between 1000 and 2000.

In one embodiment of this invention, an average molecular weight of the PEG is between 1500 and 2000.

In one embodiment of this invention, an average molecular weight of the PEG is smaller than or equal to 1000.

In one embodiment of this invention, the immersing step is performed for 30 min to 60 min.

In one embodiment of this invention, the immersing step comprises physical treatment.

In one embodiment of this invention, the washing step is performed for 3 days to 5 days.

In one embodiment of this invention, the amphipathic molecule is biocompatible.

In one embodiment of this invention, the tissue is tissue selected from the group consisting of blood vessels, blood vessel-like tissue, cardiac valves, pericardia, dura mater, corneas, and bones.

In one embodiment of this invention, the tissue is derived from a mammal.

In one embodiment of this invention, the tissue is derived from a human.

In one embodiment of this invention, the method further comprises 4) performing chemical treatment.

In one embodiment of this invention, the chemical treatment is performed with DNase.

In one embodiment of this invention, the chemical treatment is performed with DNaseI.

In one embodiment of this invention, the method further comprises disseminating a cell.

According to another aspect of the present invention, a decellularized tissue is provided, which is obtained by the above-described method.

According to another aspect of the present invention, a tissue regeneration method is provided, which comprises the steps of: a) providing decellularized tissue into an organism, wherein a cell survival rate of the tissue is less than a level at which an immune reaction is elicited in an organism; the tissue is not damaged to such an extent that hinders the tissue from exhibiting a function which was possessed by the tissue when the tissue was not decellularized; and b) incubating the tissue within the organism for a time sufficient for the tissue to regenerate.

In one embodiment of this invention, the method further comprises providing a cell to the decellularized tissue.

In one embodiment of this invention, the cell is derived from the organism.

In one embodiment of this invention, the cell is present within the organism.

In one embodiment of this invention, the cell is derived from a host homologous to the organism.

In one embodiment of this invention, the cell is derived from a host heterologous to the organism.

In one embodiment of this invention, the cell is previously isolated from the organism.

In one embodiment of this invention, the cell is a blood vessel cell or a blood vessel-like cell.

In one embodiment of this invention, the method further comprises providing a physiologically active substance capable of inducing cell differentiation to the organism.

In one embodiment of this invention, the physiologically active substance is derived from or foreign to the organism.

In one embodiment of this invention, the physiologically active substance is provided in a form of a nucleic acid or a polypeptide.

In one embodiment of this invention, the physiologically active substance is selected from the group consisting of HGF, VEGF, FGF, IGF, PDGF, and EGF.

In one embodiment of this invention, the tissue is tissue selected from the group consisting of blood vessels, blood vessel-like tissue, cardiac valves, pericardia, dura mater, corneas, and bones.

According to another aspect of the present invention, a method of producing a tissue graft is provided, which comprises the steps of: a) providing decellularized tissue into an organism, wherein a cell survival rate of the tissue is less than a level at which an immune reaction is elicited in an organism; the tissue is not damaged to such an extent that hinders the tissue from exhibiting a function which was possessed by the tissue when the tissue was not decellularized; and b) causing a self cell in the organism to infiltrate the decellularized tissue; and c) incubating the tissue within the organism for a time sufficient for the cell to differentiate.

In one embodiment of this invention, the cell is a blood vessel cell or a blood vessel-like cell.

In one embodiment of this invention, the tissue is tissue selected from the group consisting of blood vessels, blood vessel-like tissue, cardiac valves, pericardia, dura mater, corneas, and bones.

In one embodiment of this invention, the decellularization tissue comprises a cell.

In one embodiment of this invention, the decellularization tissue is autologous.

In one embodiment of this invention, the decellularization tissue is derived from a homologous host.

In one embodiment of this invention, the decellularization tissue is derived from a heterologous host.

In one embodiment of this invention, the cell is autologous.

In one embodiment of this invention, the cell is derived from a homologous host.

In one embodiment of this invention, the cell is derived from a heterologous host.

In one embodiment of this invention, the method further comprises d) providing a physiologically active substance capable of inducing differentiation of the cell.

In one embodiment of this invention, the physiologically active substance is a cytokine having hematopoiesis activity.

According to another aspect of the present invention, a tissue graft is provided, which is produced by the above-described method.

According to another aspect of the present invention, a method of treating a subject requiring implantation of tissue or an organ or treating a subject at a risk of implantation of tissue or an organ for prophylaxis, is provided, which comprises the steps of: 1) providing decellularized tissue, wherein a cell survival rate of the tissue is less than a level at which an immune reaction is elicited in an organism; the tissue is not damaged to such an extent that hinders the tissue from exhibiting a function which was possessed by the tissue when the tissue was not decellularized, or a tissue graft comprising the decellularized tissue; and 2) implanting the decellularized tissue or tissue graft to the subject.

In one embodiment of this invention, the tissue further comprises a cell.

In one embodiment of this invention, the cell is a recipient-derived cell.

In one embodiment of this invention, the cell is derived from an organism of the same species as the tissue.

In one embodiment of this invention, the tissue comprises no cell.

In one embodiment of this invention, the tissue is derived from the subject.

In one embodiment of this invention, the tissue is tissue selected from the group consisting of blood vessels, blood vessel-like tissue, cardiac valves, pericardia, dura mater, corneas, and bones.

In one embodiment of this invention, the subject is a mammal.

In one embodiment of this invention, the subject is a human.

According to another aspect of the present invention, a medicament for organ implantation is provided, which comprises A) decellularized tissue, wherein a cell survival rate of the tissue is less than a level at which an immune reaction is elicited in an organism; the tissue is not damaged to such an extent that hinders the tissue from exhibiting a function which was possessed by the tissue when the tissue was not decellularized, or a tissue graft comprising the decellularized tissue.

In one embodiment of this invention, the tissue is tissue selected from the group consisting of blood vessels, blood vessel-like tissue, cardiac valves, pericardia, dura mater, corneas, and bones.

In one embodiment of this invention, the cell is derived from a mammal.

In one embodiment of this invention, the tissue is derived from a human.

In one embodiment of this invention, the tissue is derived from the subject requiring implantation.

In one embodiment of this invention, the tissue is derived from a swine.

According to another aspect of the present invention, use of decellularized tissue is provided, in which a cell survival rate of the tissue is less than a level at which an immune reaction is elicited in an organism; the tissue is not damaged to such an extent that hinders the tissue from exhibiting a function which was possessed by the tissue when the tissue was not decellularized, or a tissue graft comprising the decellularized tissue, for producing a medicament for organ implantation.

BRIEF DESCRIPTION OF THE DRAWINGS

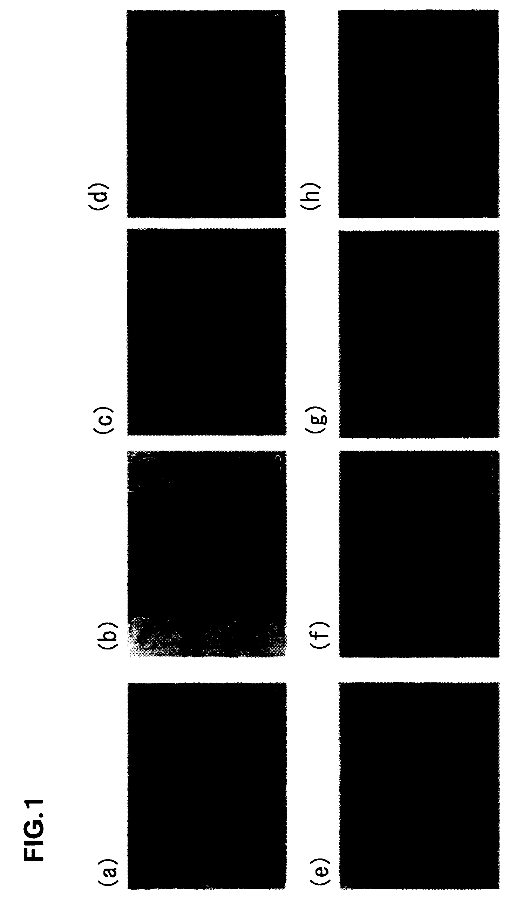

Figure **1** shows histological evaluation of decellularized tissue by hematoxylin and eosin staining (H&E staining). **(a)** and **(b)** show untreated rat aortas. **(c)** shows a PEG-treated Rat aorta. **(d)** shows a PEG/DNaseI-treated Rat aorta. **(e)** shows an untreated porcine artery. **(f)** shows a PEG-treated porcine artery. **(g)** shows a PEG/DnaseI-treated porcine artery. **(h)** shows a micellar Triton X-treated porcine artery.

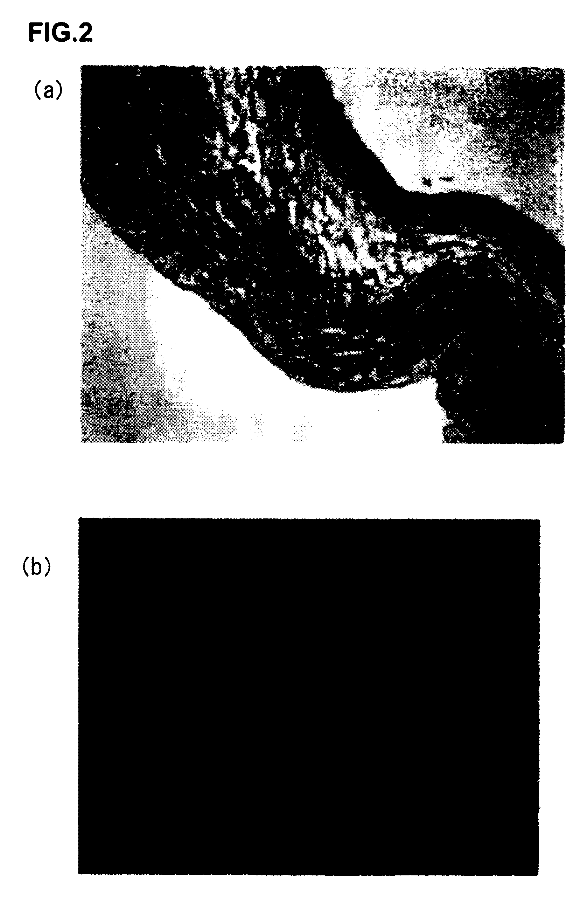

Figure **2** shows micrographs ($\times$ 10) (**(a)** and **(b)**) of the distribution of cell nuclei stained by H&E staining in porcine aortic valve tissue and wall tissue before decellularization. A difference is seen between cusp and wall structures. The cusp was thin and was not made of dense connective tissue. Therefore, it was relatively easy to remove cells from the cusp. In contrast, the wall tissue was made of complicated elastic layers. Substances were hindered from diffusing into the inside and outside of the tissue. **(a)** shows a porcine aortic cusp, while **(b)** shows a porcine aorta.

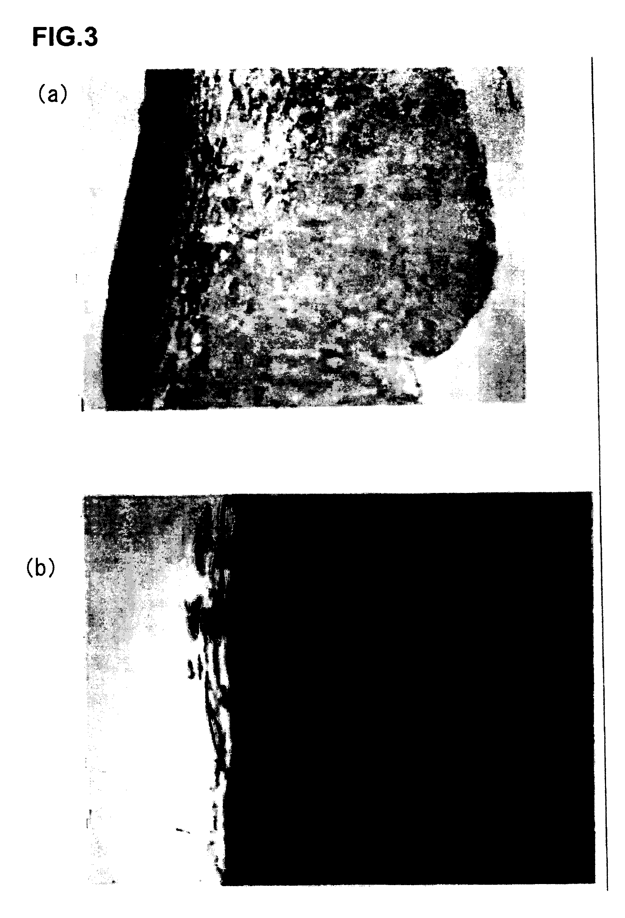

Figure **3** shows micrographs (×10) ((**a**) and (**b**)) of cusp and wall tissue decellularized by a first generation process. In the cusp, substantially no nucleus is seen in extracellular matrices. In the aorta wall, cells and nuclei are confirmed in a middle portion (1/3 portion). (**a**) shows the porcine aortic valve cusp decellularized by the first generation process, which was stained by H&E staining. Ventricle surface layers are shown on the right side, while fibrous layers are shown on the left side. (**b**) shows the porcine aorta decellularized by the first generation process, which was stained by H&E staining. It is seen that nuclei are present in a middle portion of the aorta wall, while no nuclei are present at the right end of the calcification surface.

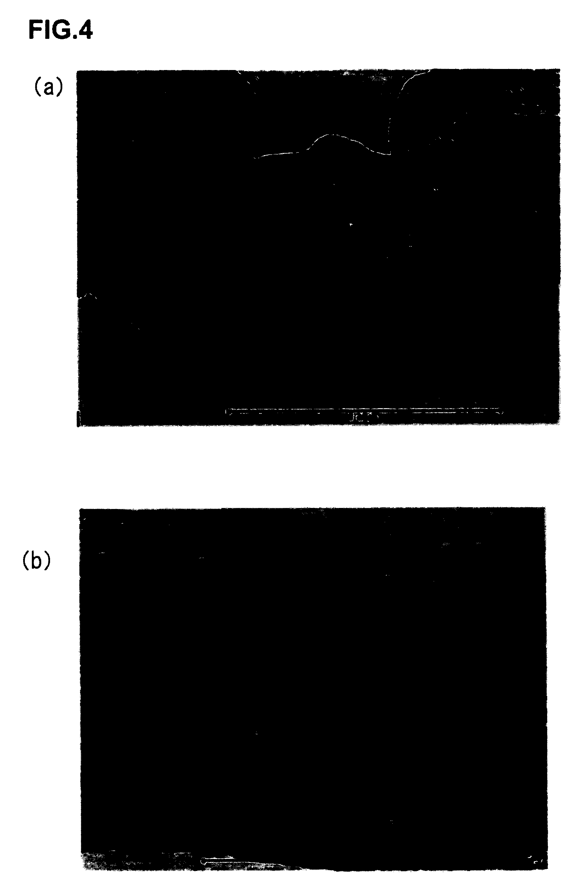

Figure **4** shows micrographs (×10) ((**a**) and (**b**)) of the cusp and aorta wall tissue decellularized by the first generation process. In the cusp, no cells are seen. In the aorta wall, a basophilic staining substance is only sparsely present (considered to be disrupted chromatin). (**a**) shows the porcine aortic valve cusp decellularized by the first generation process, which was stained by H&E staining. (**b**) shows the porcine aorta decellularized by the first generation process, which was stained by H&E staining. In the porcine aorta, nucleus debris is slightly seen.

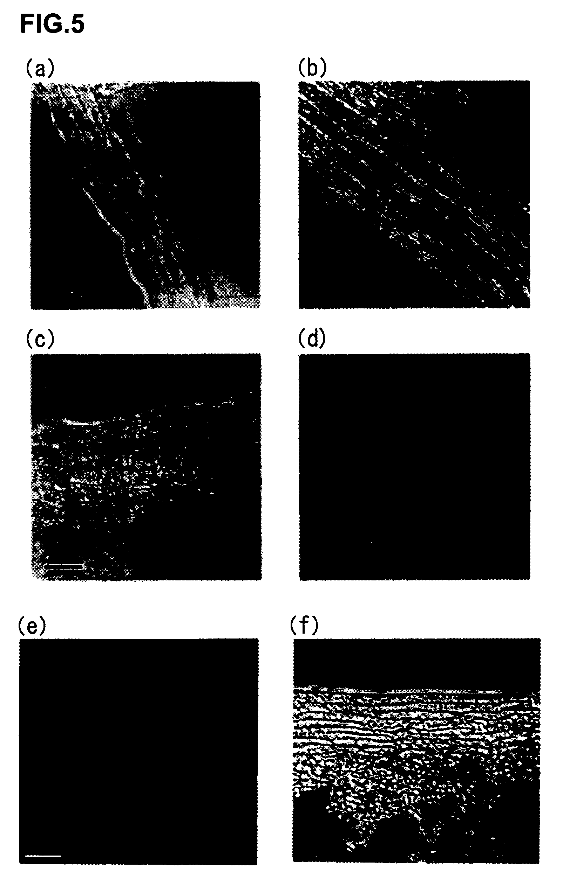

Figure **5** shows residual matrices of Type I collagen ((**a**) and (**b**)) and Type IV collagen ((**c**) and (**d**)) in decellularized tissue. (**a**) and (**c**), and (**b**) and (**d**) are micrographs taken by fluorescent and transmissive confocal microscopes, respectively, of Type I and IV collagen. (**e**) and (**f**) show the dissemination of DiI-labeled ECs in decellularized tissue. (**a**) and (**c**) show untreated rat aortas. (**b**) and (**d**) show PEG-treated rat aortas. (**e**) shows the internal surface of the blood vessel. (**f**) shows a section (major axis) (the above side is the inner portion).

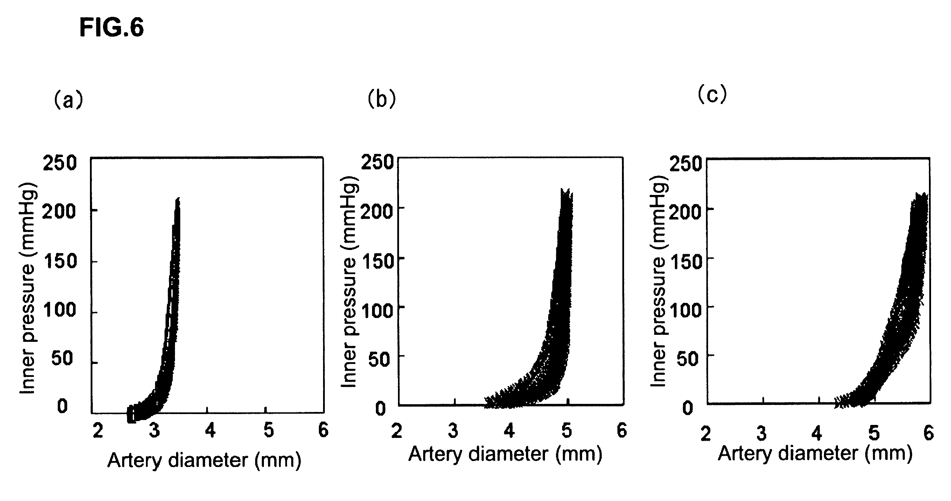

Figure **6** shows P-D relationship between a treated porcine artery and an untreated porcine artery. (**a**) indicates an untreated porcine artery. (**b**) indicates a PEG/DNaseI-treated porcine artery. (**c**) indicates a porcine artery treated with Triton (registered trademark) X100. In (**a**), (**b**), and (**c**), the x axis represents the diameter (mm) of a blood vessel, while the y axis represents load pressure (mmHg).

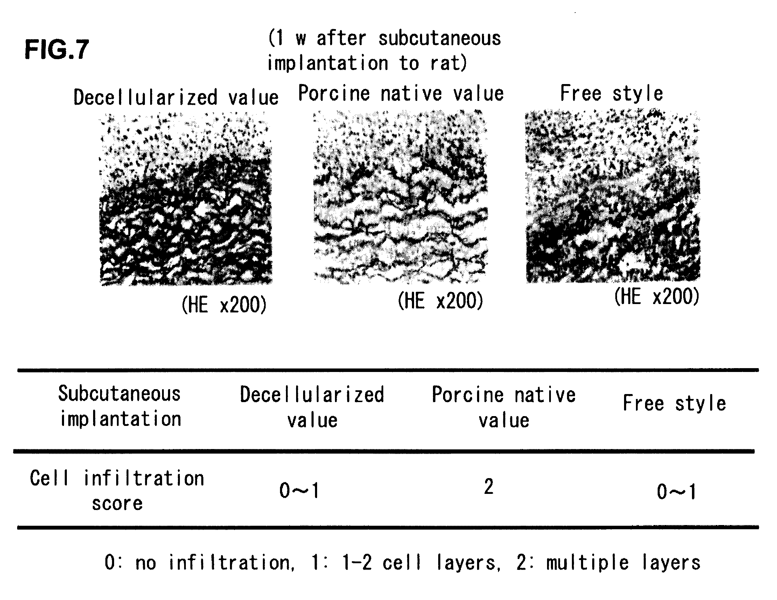

Figure **7** shows micrographs of decellularized tissue (valve) implanted under rat skin, which was stained by H&E staining one week after implantation. The left micrograph shows a decellularized valve. The middle micrograph shows a porcine native valve. The right micrograph shows a Free Style valve as a control. A table showing relative cellular infiltration scores is shown at the bottom of Figure **7**. The decellularized tissue (valve) and the Free Style valve had a score of 0-1 (i.e., substantially no infiltration was seen), while the other porcine tissue valve had a score of 2 (i.e., infiltration was seen in a plurality of layers).

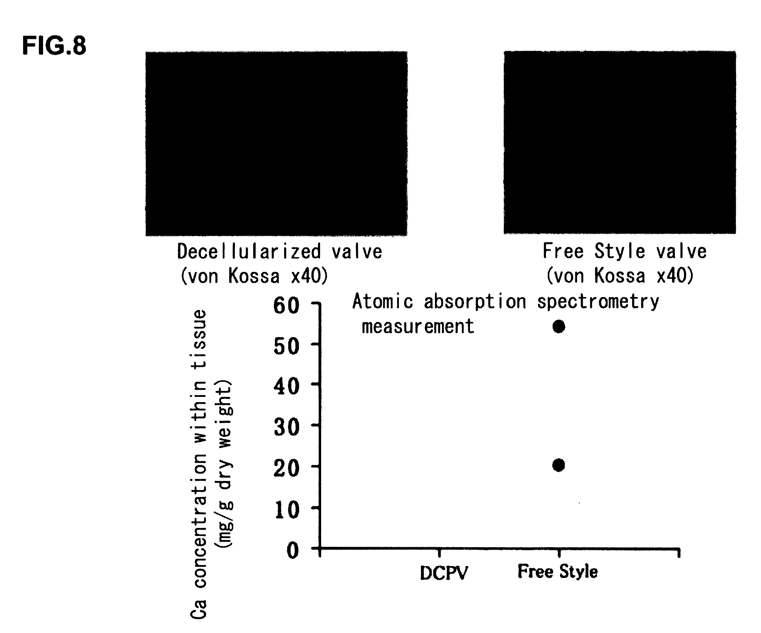

Figure **8** shows micrographs (×40) of calcification. Calcification was determined by von Kossa staining. A micrograph at the upper left portion shows the calcification of decellularized tissue of the present invention (valve). A micrograph at the right portion shows the calcification of a Free Style valve as a control. A graph at the lower portion shows a result of atomic absorption spectrometry measurement of calcium concentration in the tissue. DCPV indicates the decellularized tissue of the present invention, while Free Style indicates the Free Style valve (available from Medtronic) as a control.

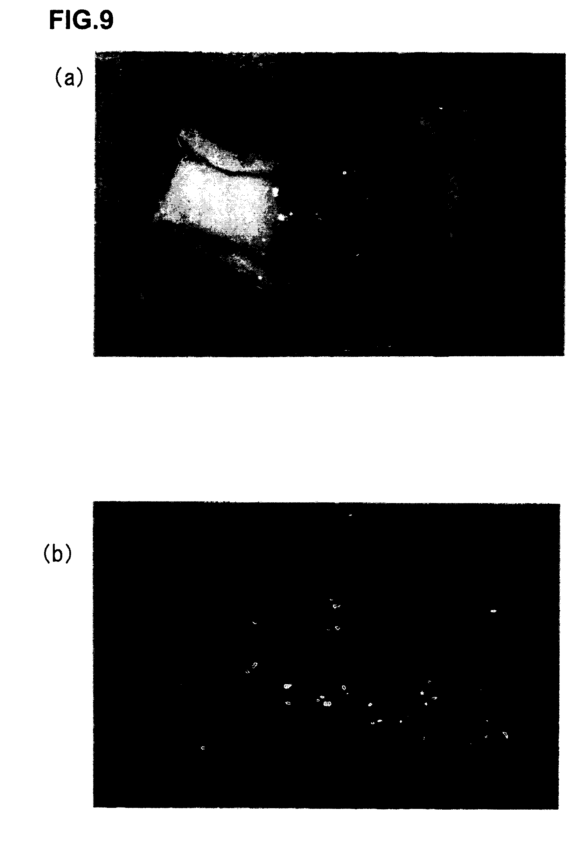

Figure **9** shows photographs of aorta wall grafts after implantation into dogs. A portion of aorta wall tissue treated by the first generation decellularization process was implanted into a dog descending aorta. The graft was removed from the dog which died after two weeks. (**a**) is a photograph of autopsy of the dog thoracic descending aorta implanted with the porcine aorta wall graft, viewed from the lumen side. (**b**) is a photograph of autopsy of the excised sample viewed from the outer membrane side. Note that the dog implanted with decellularized tissue obtained by the decellularized process of the present invention survived and was healthy 4 months after implantation. At that time, the dog was sacrificed. It was found that the aorta wall graft was normally regenerated.

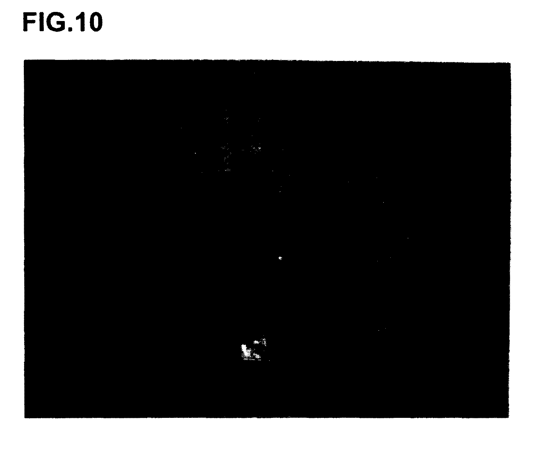

Figure **10** shows a photograph after autopsy in a second dog implantation experiment, showing the outer membrane side of a dog thoracic descending aorta (wall) implanted with a portion of a porcine aortic valve decellularized by the first generation process. The graft was removed from the dog which died after two weeks. The autopsy sample photograph shows the aneurismal extension of an aorta wall.

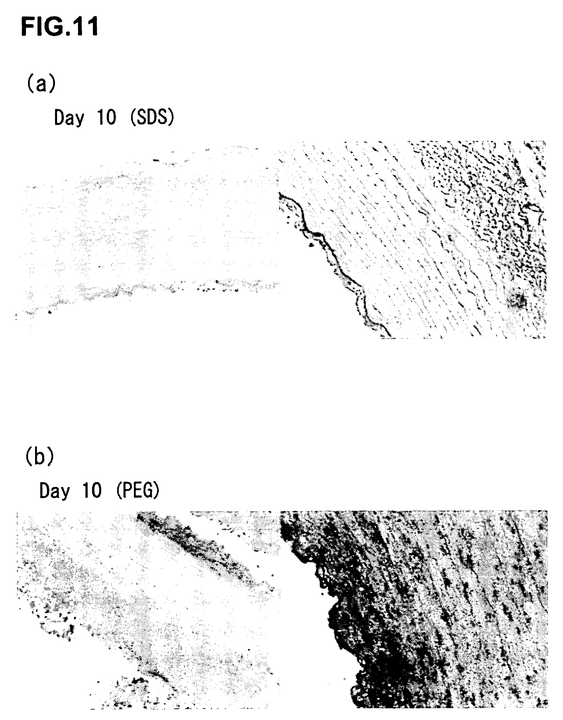

Figure **11** shows photographs of cell replacement. (**a**) shows cell replacement in decellularized tissue obtained by conventional SDS treatment. It is seen that substantially no cell replacement occurred. (**b**) shows cell replacement in decellularized tissue obtained by PEG treatment of the present invention. It is seen that substantial cell replacement occurred only 10 days after implantation.

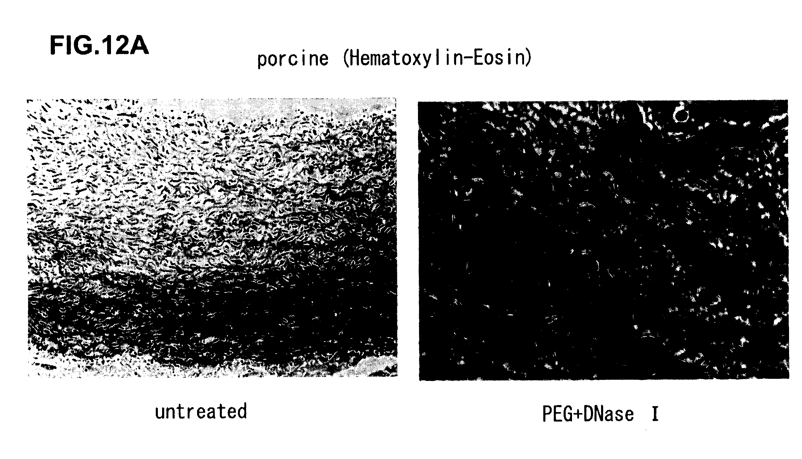

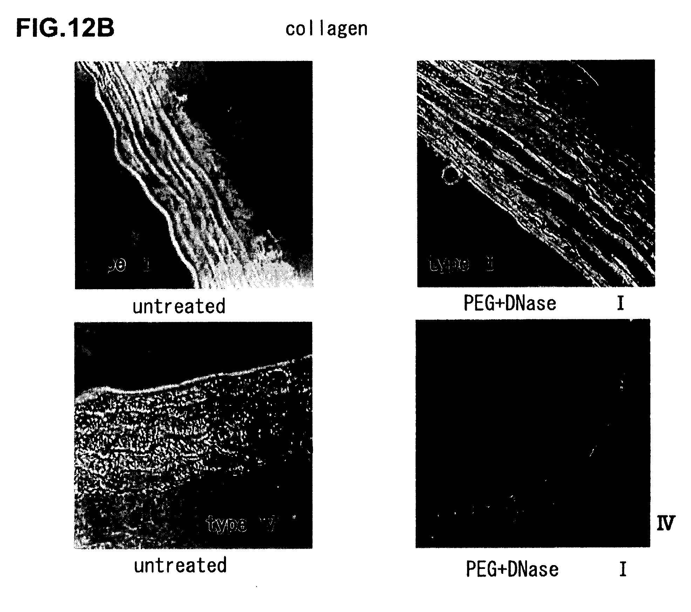

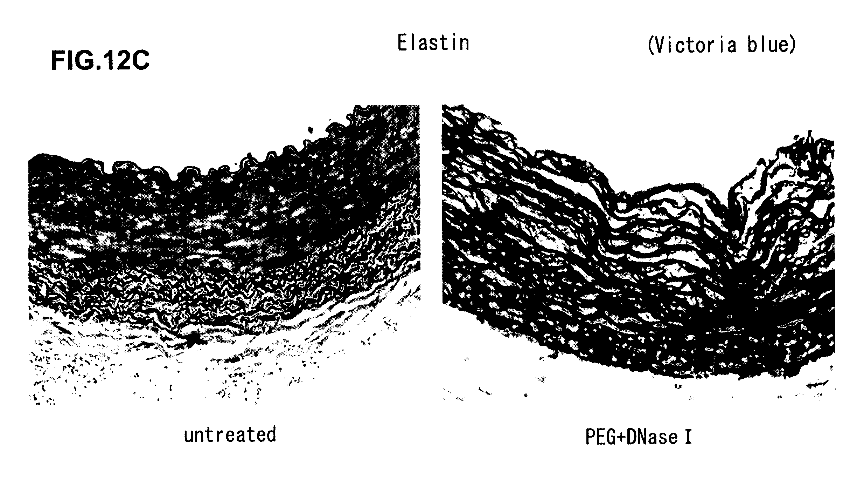

Figures **12A** to **12C** show comparison of decellularized porcine tissue according to an illustrative embodiment of the present invention and untreated tissue. Figure **12A** is a photograph showing hematoxylin-eosin staining. Figure **12B** is a photograph showing the presence of collagen. Figure **12C** is a photograph showing the presence of elastin.

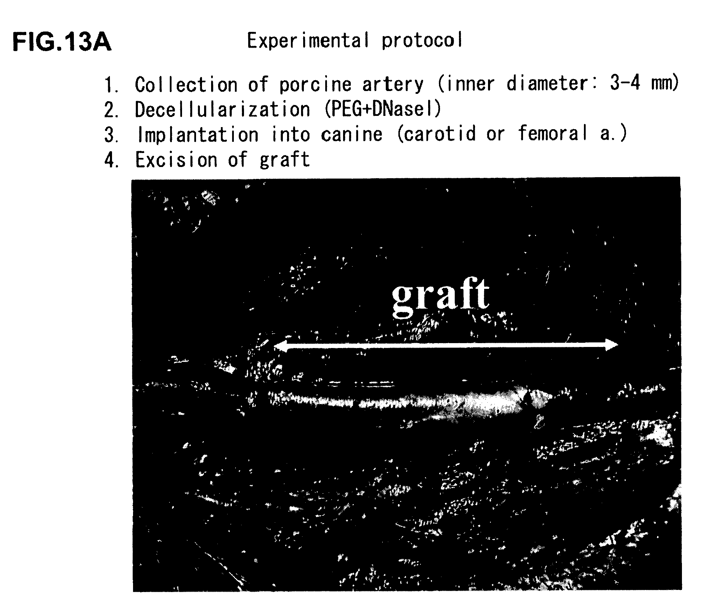

Figure **13A** shows an example of implantation of a graft (here, a blood vessel).

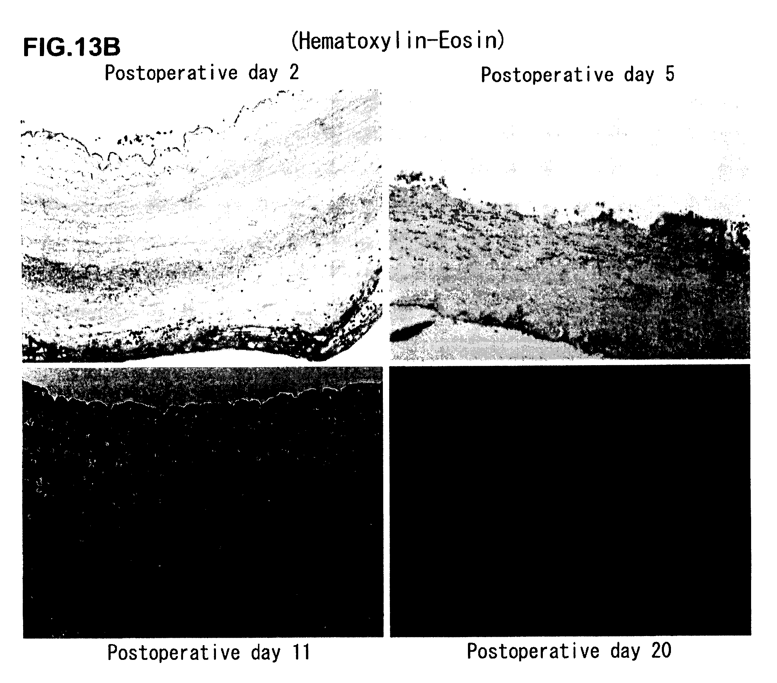

Figure **13B** shows decellularized tissue of the present invention after implantation. Postoperative cellular infiltration is seen.

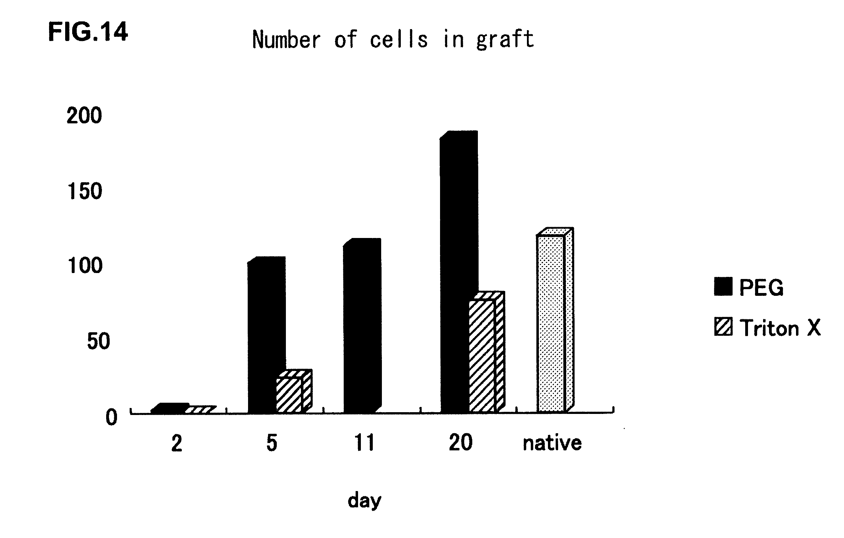

Figure **14** shows the number of cells counted in the experiment shown in Figure **13B.**

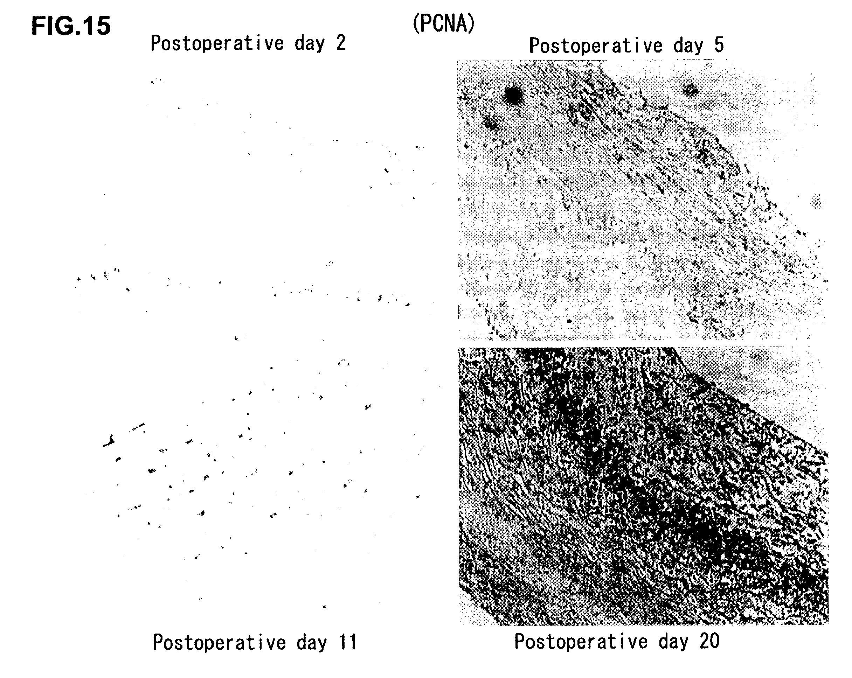

Figure **15** shows PCNA (proliferative cell nucleus antigen) in decellularized tissue of the present invention after implantation.

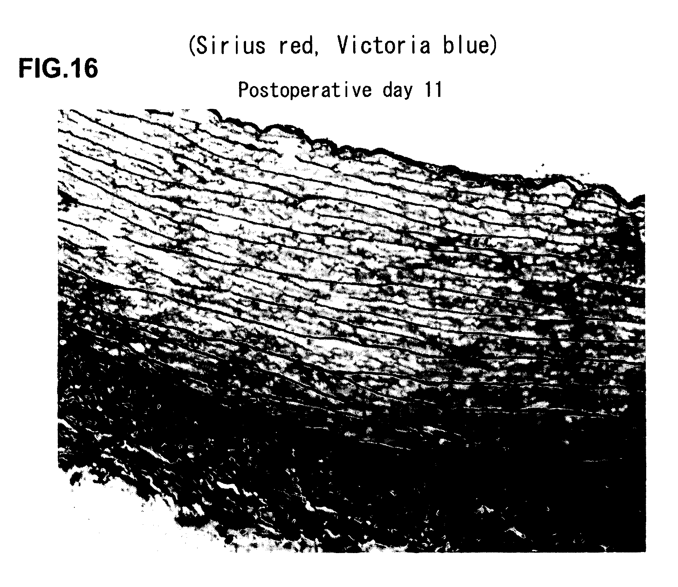

Figure **16** shows decellularized tissue of the present invention on day 11 after implantation, stained by sirius red and victoria blue staining.

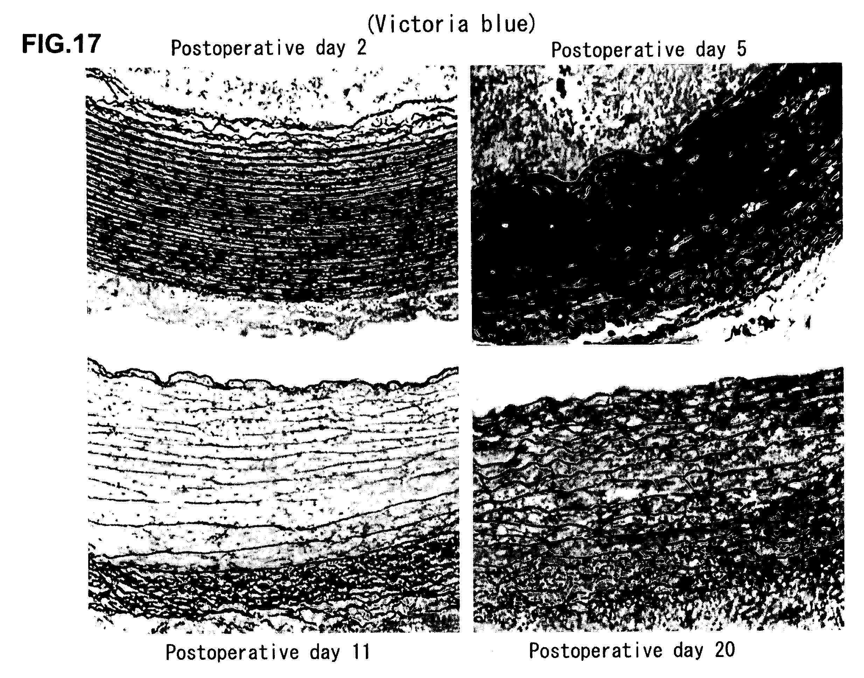

Figure **17** shows decellularized tissue of the present invention after implantation, stained by victoria blue staining.

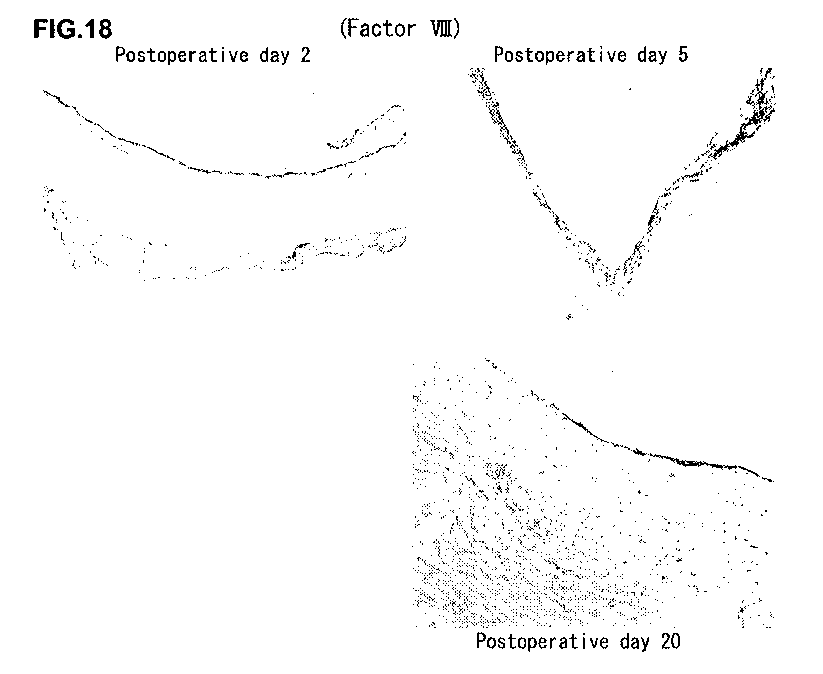

Figure **18** shows the behavior of factor VIII in decellularized tissue of the present invention after implantation.

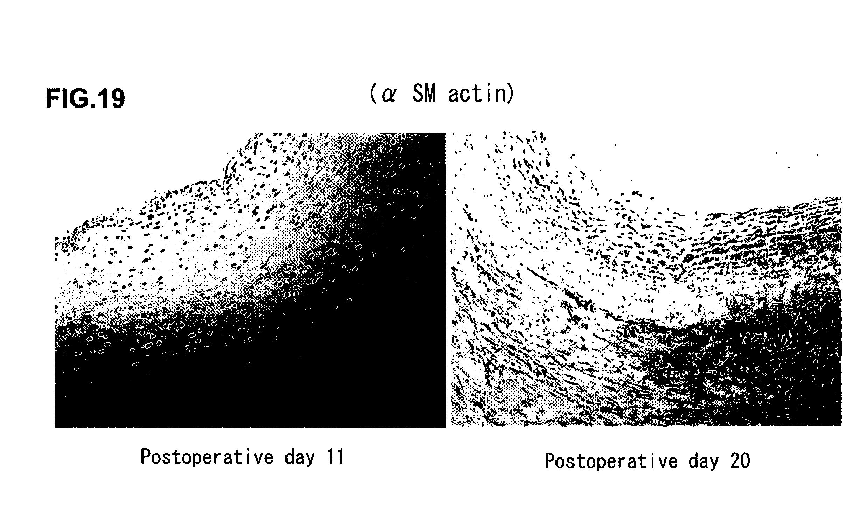

Figure **19** shows the behavior of α-SM actin in decellularized tissue of the present invention after implantation.

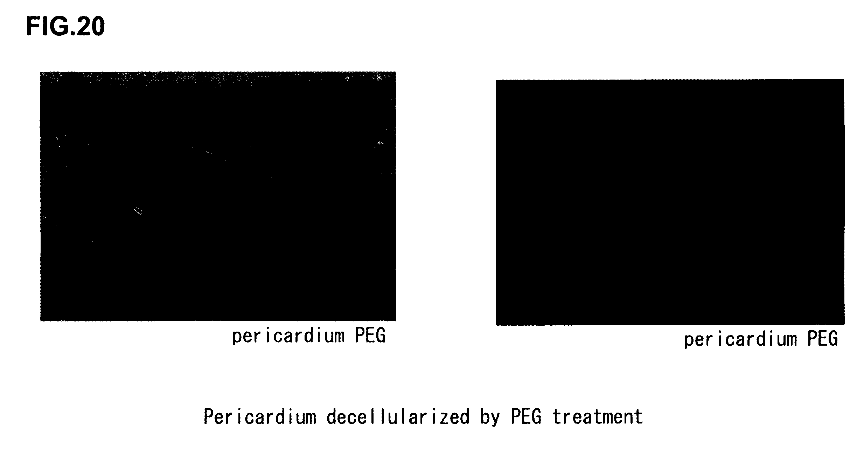

Figure **20** shows a pericardium decellularized by polyethylene glycol treatment.

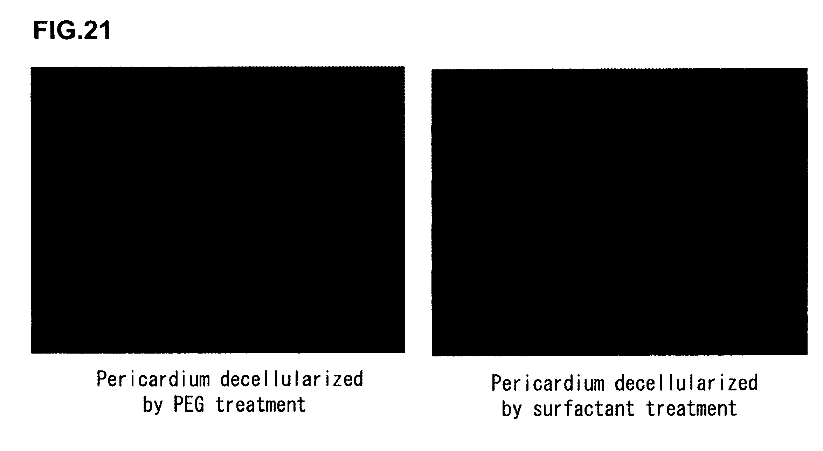

Figure **21** shows a comparison between a pericardium decellularized by polyethylene glycol treatment and a pericardium decellularized by surfactant treatment.

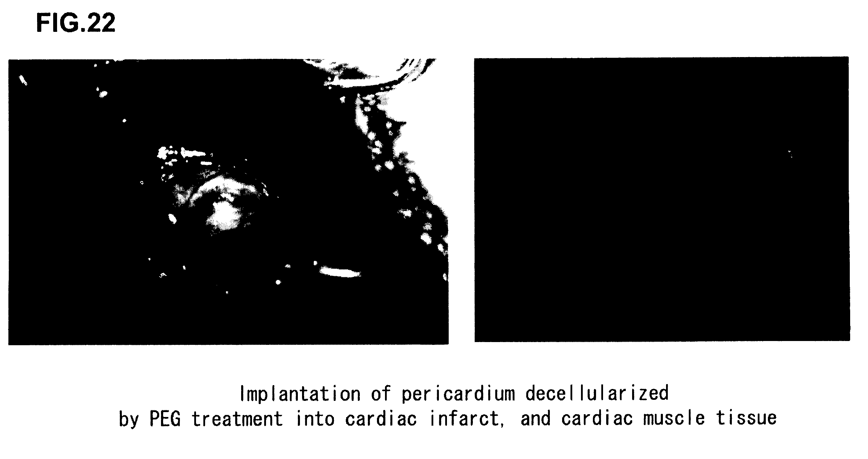

Figure **22** shows implantation of a pericardium decellularized by polyethylene glycol treatment into cardiac infarct, and cardiac muscle tissue.

DETAILED DESCRIPTION OF THE INVENTION

It should be understood throughout the present specification that expression of a singular form includes the concept of their plurality unless otherwise mentioned. Specifically, articles for a singular form (e.g., "a", "an", "the", etc. in English; "ein", "der", "das", "die", etc. and their inflections in German; "un", "une", "le", "la", etc. in French; "un", "una", "el", "la", etc. in Spanish, and articles, adjectives, etc. in other languages) include the concept of their plurality unless otherwise mentioned. It should be also understood that the terms as used herein have definitions typically used in the art unless otherwise mentioned. Thus, unless otherwise defined, all scientific and technical terms have the same meanings as those generally used by those skilled in the art to which the present invention pertain. If there is contradiction, the present specification (including the definition) precedes.

(Definition of Terms)

The definitions of terms used herein are described below.

(Regeneration Medicine)

As used herein, the term "regeneration" refers to a phenomenon in which when an individual organism loses a portion of tissue, the remaining tissue grows and recovers. The extent or manner of regeneration varies depending among animal species or among tissues in the same individual. Most human tissues have limited regeneration capability, and therefore, complete regeneration is not expected if a large portion of tissue is lost. In the case of heavy damage, tissue having strong proliferation capability different from that of lost tissue may grow, resulting in incomplete regeneration where the damaged tissue is incompletely regenerated and the function of the tissue cannot be recovered. In this case, a structure made of a bioabsorbable material is used to prevent tissue having a strong proliferation capability from infiltrating the defective portion of the tissue so as to secure space for proliferation of the damaged tissue. Further, by supplementing with a cell growth factor, the regeneration capability of the damaged tissue is enhanced. Such a regeneration technique is applied to cartilages, bones, and peripheral nerves, for example. It has been so far believed that nerve cells and cardiac muscles have no or poor regeneration capability. Recently, it was reported that there are tissue stem cells (somatic stem cells) which have both the capability of differentiating into these tissues and self-proliferation capability. Expectations are running high for regeneration medicine using tissue stem cells. Embryonic stem cells (ES cells) are cells which have the capability of differentiating into all tissues. Efforts have been made to use ES cells for regeneration of complicated organs, such as kidney, liver, and the like, but have not yet been realized.

As used herein, the term "cell" is defined as having the widest meaning used in the art, referring to a structural unit of multicellular organisms, which has an enveloping membrane structure for separating the cell from the outside, has self-regeneration capability, and which is a living body having genetic information and an expression mechanism. In the method of the present invention, any cell can be used as a subject. The number of cells used in the present invention can be counted through an optical microscope. When counting using an optical microscope, the number of nuclei is counted. Tissues are sliced into tissue sections, which are then stained with hematoxylin-eosin (HE) to variegate nuclei derived from extracellular matrices (e.g., elastin or collagen) and cells. These tissue sections are observed under an optical microscope and the number of nuclei in a particular area (e.g., 200 $\mu$mm $\times$ 200 $\mu$m) can be estimated to be the number of cells.

Cells may elicit calcification and immune reactions. Therefore, non-self cells should be removed as much as possible for implantation of tissue or organs. In the case of self cells, decellularization is not required, since no immunological rejection problem is usually raised. However, since decellularization is sometimes preferable, cells should also be removed as much as possible. There is a strong desire for decellularized tissue.

Asusedherein, the term "cell replacement" indicates that cells originally existing are replaced with other infiltrating cells in decellularized tissue. This term is also referred to as "cellular infiltration". Preferably, in the present invention, cell replacement is carried out with cells of a host undergoing implantation. It was confirmed that after implantation, host-derived cells infiltrated and replaced decellularized tissue and grafts prepared with the technique of the present invention. Such an event never occurred in conventionally prepared tissue grafts. This finding per se can be said to indicate an unexpected, excellent effect of the present invention.

As used herein, the term "tissue" refers to a group of cells having the same function and form in cellular organisms. In multicellular organisms, constituent cells are usually differentiated so that the cells have specialized functions, resulting in division of labor. Therefore, multicellular organisms are not simple cell aggregations, but constitute organic or social cell groups having a certain function and structure. Examples of tissue include, but are not limited to, integument tissue, connective tissue, muscular tissue, nervous tissue, and the like. Tissue targeted by the present invention may be derived from any organ or part of an organism. In a preferred embodiment of the present invention, tissue targeted by the present invention includes, but is not limited to, blood vessels, blood vessel-like tissue, cardiac valves, pericardia, dura mater, corneas, and bone tissue. Since non-micellar amphipathic molecules used in the present invention acts in a mechanism like cell component extraction, tissue derived from any organ can be in principle treated with the method of the present invention.

As used herein, "membrane-like tissue" is also referred to as "planar tissue" and refers to a tissue having a membrane form. Membrane-like tissue includes tissue from organs, such as pericardia, dura mater, and corneas.

As used herein, "organ" or "part" is used interchangeably, referring to a structure which is a specific portion of an individual organism where a certain function of the individual organism is locally performed and which is morphologically independent. Generally, in multicellular organisms (e.g., animals and plants), organs are made of several tissues in specific spatial arrangement and tissue is made of a number of cells. Examples of organs or parts include organs or parts related to a blood vessel system. In one embodiment, examples of organs targeted by the present invention include ischemic organs (the heart undergoing cardiac infarction, skeletal muscle undergoing ischemia, and the like). In one preferred embodiment, organs targeted by the present invention are blood vessels, blood vessel-like tissue, cardiac valves, pericardia, dura mater, corneas, and bones. In another preferred embodiment, organs targeted by the present invention are cardiac valves, pericardia, and blood vessels.

In order to prepare grafts suitable for implantation, it may be optionally desirable that organs are cultured. Organ culture refers to *in vitro* culture of the whole or part of an embryonic or mature organ extracted from an organism where the structure, function and differentiation thereof are maintained. In contrast, general tissue culture mainly aims at cellproliferation, where dedifferentiation is likely to occur. Organ culture is conducted by a method, including, but not limited to, the watch glass method, the block Petri dish method, the support method, the sponge matrix method, the Trowell method, and the like. Also, large-scale cell culture (e.g., the hollow fiber method, the fixed bed method, the spin filter method, the precipitation tank method, perfusion culture, circulation type organ culture, etc.) as described in Tanpakushitsu·K-akusan·Koso [Protein·Nucleic acid · Enzyme] 45, 2188-2200(2000), may be used. An organ or tissue prepared by the decellularization method of the present invention is placed in a culture medium which is supplemented with various substances so as to regulate the structure, function, growth and differentiation, organ-to-organ interaction, and the like.

As used herein, the term "decellularization" and "decellularize" refers to removal of cells from tissue or organs. Preferably, decellularization may be carried out without damage to the structure and function of the original tissue or organs. Plasma components, cytosol components, cytoskeletons, and cell membrane components are removed from decellularized tissue. However, components required for maintaining the structure of tissue, such as extracellular matrix (ECM) components (e.g., elastin, collagen (Type I, IV), laminin, and the like), are maintained without degeneration. Therefore, decellularized tissue or organs are preferably substantially equivalent to untreated tissue or organs in terms of properties thereof, such as shape, physical strength, elasticity, flexibility, and the like. Since extracellular matrices are not degenerated after implantation, it is preferable to provide an environment suitable for the infiltration, adhesion, proliferation, and expression and maintenance of differentiation traits, of cells of recipients, so that the matrices are replaced with tissue made of self cells. The extent of decellularization can be measured using a cell survival rate as an index.

MHC class I and II proteins are preferably removed from decellularized tissue. MHC class I and II proteins can be confirmed by SDS PAGE and/or western blotting analysis. Other extracellular matrix proteins can be confirmed by SDS PAGE and/or western blotting analysis. Structural proteins in cells (collagen, elastin, and the like) can be evaluated by amino acid analysis (e.g., Edman degradation, automatic analysis using a peptide analyzer available from PE Biosystems, or the like). As a result, the influence of decellularized processes on ECM can be determined. Lipids and phospholipids contained in cell membrane and cells can be analyzed using thin layer chromatography and HPLC. Sugar chains (e.g., glycosaminoglycans or the like) can be analyzed by agarose gel electrophoresis or the like. This analysis can analyze the glycosaminoglycan composition of extracellular matrices as well as the presence of $\alpha$-Gal or the like.

As used herein, "cell survival rate" refers to a ratio of survival cells after decellularization of tissue by the method of the present invention to cells originally existing in the tissue. As used herein, the cell survival rate is typically measured by a counting method using a microscope after hematoxylin and eosin staining (H&E staining). This method comprises counting nuclei variegated by HE staining under an optical microscope. Therefore, for example, this method comprises the following steps: variegating samples by HE staining; counting the number of nuclei (the number of cells) present in an area of 100 mm $\times$ 100 mm in the sample; optionally repeating the counting step (e.g., a total of 8 times) and averaging the counts; and calculating a ratio to a control (e.g., untreated tissue is regarded as 100%). The ratio of the control (e.g., a ratio of survival nuclei to untreated tissue) can be regarded as a cell survival rate. Therefore, in this case, the cell survival rate (%) = (the number of nuclei in treated tissue)/(the number of nuclei in untreated tissue) $\times$ 100. The cell survival rate can also be determined by measuring the amount of survival DNA. This is because the total amount of DNA in cells of tissue is known to be generally proportional to the number of the cells of the tissue. Methods for measuring DNA are known in the art. For example, the amount of DNA extracted from tissue can be determined using a fluorescent reagent, such as Hoechst 33258 (from Molecular Probes) or the like. Specifically, tissue is solated by ultrasonic pulverization treatment or the like; and the amount of DNA in extract supernatant can be determined by reacting with Hoechst 33258 (Ex (Excitation wavelength) 356, EM (Emission wavelength) 492) capable of specifically binding to DNA components in buffer solution and emitting fluorescence, and measuring the strength of fluorescence. The term "less than a level which can elicit an immune reaction in organisms" of the cell survival rate of certain tissue indicates that when the tissue is implanted into organisms, no immune reaction is elicited for a certain period of time (e.g., several months to several years, preferably the entirety of the rest of life).

As used herein, the term "immune reaction" refers to a reaction due to loss of coordination of immunologic tolerance between a graft and a host, including, for example, hyperacute rejection reactions (within several minutes after implantation; immune reactions due to an antibody, such as $\beta$-Gal or the like), acute rejection reactions (cell-mediated immune reactions about 7 to 21 days after implantation), chronic rejection reactions (rejection reactions due to cell-mediated immune response after three months), and the like.

As used herein, elicitation of immune reactions can be determined by histological examination of the type, number, or the like, of cells (immune cells) infiltrating implanted tissue by observing under a microscope tissue sections stained by HE staining or immunological staining.

As used herein, the term "calcification" refers to precipitation of calcareous substances in organisms.

As used herein, "calcification" *in vivo* can be determined by measuring calcium concentration. Specifically, implanted tissue is taken out; the tissue section is dissolved by acid treatment or the like; and the atomic absorption of the solution is measured by a trace element quantifying device.

As used herein, the term "within organism(s) (or in organism(s))" or "*in vivo*" refers to the inner part of organism(s). In a specific context, "within organism(s)" refers to a position at which a subject tissue or organ is placed.

As used herein, "*in vitro*" indicates that a portion of an organism is extracted or released outside the organism for various purposes of research (e.g., in a test tube). The term *in vitro* is in contrast to the term *in vivo*.

As used herein, the term "*ex vivo*" refers to a series of operations where target cells into which a gene will be introduced are extracted from a subject; a therapeutic gene is introduced *in vitro* into the cells; and the cells are returned into the same subject.

As used herein, the term "a function in the untreated form" of certain tissue refers to a function possessed *in vivo* by the tissue in its normal state. Therefore, for example, in the case of cardiac valves, cardiac valves usually have a function of preventing a back flow of blood from a ventricle to an atrium or from the pulmonary artery and aorta to an atrium, a function in the untreated form of cardiac valves refers to the function of preventing a back flow of blood from a ventricle to an atrium or from the pulmonary artery and aorta to an atrium.

As used herein, the term "extracellular matrix" (ECM) refers to a substance existing between somatic cells no matter whether the cells are epithelial cells or non-epithelial cells. Extracellular matrices are involved in supporting of tissue as well as internal environmental structure essential for survival of all somatic cells. Extracellular matrices are generally produced from connective tissue cells. Some extracellular matrices are secreted from cells possessing basal membrane, such as epithelial cells or endothelial cells. Extracellular matrices are roughly divided into fibrous components and matrices filling there between. Fibrous components include collagen fibers and elastic fibers. A basic component of matrices is a glycosaminoglycan (acidic mucopolypolysaccharide), most of which is bound to non-collagenous protein to form a polymer of a proteoglycan (acidic mucopolysaccharide-protein complex). In addition, matrices include glycoproteins, such as laminin of basal membrane, microfibril around elastic fibers, fibers, fibronectins on cell surface, and the like. Particularly differentiated tissue has the same basic structure. For example, in hyaline cartilage, chondroblasts characteristically produce a large amount of cartilage matrices including proteoglycans. In bones, osteoblasts produce bone matrices which cause calcification. One embodiment of the present invention may be characterized in that extracellular matrices (e.g., elastin, collagen (e.g., Type I, IV, and the like), and the like) are not substantially changed from before the decellularization treatment of the present invention.

As used herein, the term "tissue damage rate" refers to a parameter indicating a function of tissue or an organ, which is an index of how much tissue or an organ is damaged and damaged after treatment or an index of how much an original function of the tissue or organ can be exhibited. As used herein, a method for measuring a tissue damage rate is well known in the art. For example, the rate can be determined by counting elastin rupture sites. In a method used herein, a visual field is divided into units of 100 $\mu$m $\times$ 100 $\mu$m and the number of units having an elastin rupture site is counted. There were 24 units per visual field. In HE-stained tissue sections, extracellular matrices were counted under a microscope. A damage rate is calculated as x/24, where untreated tissue is assumed to have a damage rate of 0%, i.e., x=0.

As used herein, the "tissue strength" refers to a parameter indicating a function of tissue or an organ, which is the physical strength of the tissue or organ that is generally determined by measuring tensile strength. A general tensile testing is well known and is not herein described in detail. By analyzing data obtained by a general tensile testing, various data such as breaking strength, stiffness, Young's modulus, and the like can be obtained and can be herein used as indexes of tissue strength. As used herein, in the case of lumenal tissue, tissue strength can be represented by a stiffness parameter ($\beta$ value). A $\beta$ value is calculated by the following formula after the P-D (pressure-diameter) relationship is prepared:

$$Ln(P/Ps) = \beta(D/Ds-1) \qquad (1)$$

where Ps and Ds represent standard values at 100 mmHg and P and D represent pressure and diameter, respectively.

The opposite ends of lumenal tissue, such as a blood vessel or the like, are fixed to pipe-like units or the like and the outside and inside of the lumenal tissue are filled with physiological saline. In this situation, pressure is applied to the inside of the tissue by an external device, while monitoring the outer diameter of the tissue. The measured pressure and diameter are inserted into formula (1) to obtain a $\beta$ value (Sonoda H., Takamizawa K., et al., J. Biomed. Mater. Res. 2001:266-276).

As used herein, the term "amphipathic molecule" refers to a molecule having both a hydrophilic group (carboxy group, sulfate group, quaternary ammonium group, hydroxy group, etc.) and a hydrophobic group (also referred to as lipophilic group; including a long chain hydrocarbon group and the like). Amphipathic molecules have affinity for either polar solvents or non-polar solvents. This property is called amphiphilicity.

As used herein, the term "surfactant" refers to a substance which is dissolved in liquid and has an action to significantly reduce the surface tension of the liquid. There are a hydrophilic portion and a hydrophobic portion separately present within the molecule, so that the molecule is easily adsorbed onto a surface. Surfactant molecules form a molecular cluster, which is called micelle, at a certain concentration (critical micelle concentration) or more. Therefore, the term "surfactant" as used herein partially overlaps the term "amphipathic molecule". As used herein, the term "micelle" refers to a cluster of amphipathic molecules, such as a surfactant, formed when the molecules are dissolved in water at a certain concentration or more, in which the hydrophilic groups face the outside of the cluster while the lipophilic groups face the inside of the cluster. Formation of micelles abruptly occurs at a certain concentration, which concentration is called "critical micelle concentration". A property of an aqueous solution significantly changes at this concentration. Micelle-forming molecules are strongly absorbed onto a two-phase interface due to the hydrophilic-lipophilic balance of a surfactant. As a result, the free energy of the interface is significantly reduced so that cell components, such as proteins, lipids, and the like, are solubilized. Surfactants for decellularization are generally used at a critical micelle concentration or more (e.g., 1%). Therefore, decellularization treatment is achieved by a mechanism of decreasing free energy. Acritical micelle concentration is determined depending on the substance, and is well known or can be determined by preparing its aqueous solution.

As used herein, the term "non-micelle forming" refers to a property of an amphipathic molecule which does not form micelle at a predetermined concentration. Preferably, the property of forming no micelle may be maintained at any concentration. It is desirable that substances used in the present invention (e.g., polyethylene glycol) have no critical micelle concentration in an aqueous solution.

As used herein, the term "non-micelle forming amphipathicmolecule" refers to an amphipathic molecule which does not form micelle at a predetermined concentration. Preferably, such a molecule has no critical micelle concentration within a concentration range, in which such a molecule can exist as an aqueous solution, can be dissolved in water. Therefore, the molecule is not micellar at any concentration. When a non-micelle forming amphipathic molecule is used, decellularization treatment maybe carried out by a mechanism similar to extraction of cell components using a chemical extraction technique. Therefore, decellularization treatment using a non-micelle forming amphipathic molecule solution is different from conventional decellularization treatment using a surfactant. As a result, a significant difference occurs in the degree of tissue damage. For decellularization treatment, any non-micelle forming amphipathic molecule, preferably 1,2-epoxide polymer, and more preferably polyethylene glycol, may be herein used.

As used herein, the term "non-micellar" refers to a state of not forming micelles when an amphipathic molecule is mentioned. Such a non-micellar state may be achieved by an amphipathic molecule at less than a critical micelle concentration or a non-micelle forming amphipathic molecule. Therefore, such a non-micellar state may be achieved with 1,2-epoxide polymer (particularly, polyethylene glycol). Alternatively, a surfactant, such as SDS, Triton X-100 (registered trademark), or the like, may be used to achieve a non-micellar state when the surfactant is present at less than a critical micelle concentration, and can be used in the present invention.

As used herein , the term "1,2-epoxide polymer" refers to a polymer formed by polymerization of 1,2-epoxide as a monomer. 1,2-epoxide polymers include, but are not limited to, ethylene oxide polymers or copolymers therewith, propylene oxide polymers or copolymers therewith, and higher 1,2-epoxide polymers or copolymers therewith. 1, 2-epoxide is a compound having a structure in which oxygen atoms are bound to two carbon atoms which has been bound to each other within the molecule, i.e., an oxygen atom is bound to each of positions 1 and 2. Examples of 1,2-epoxides include, but are not limited to, ethylene oxide, propylene oxide, butylene oxide, epichlorohydrin, and the like. Examples of 1,2-epoxide polymers include, but are not limited to, ethylene oxide polymers, propylene oxide polymers, copolymers therewith, polypropylene glycol, polyethylene glycol, and the like. Preferably, 1,2-epoxide polymers include, but are not limited to, polyethylene glycol, polypropylene glycol, and the like. A more preferable 1,2-epoxide polymer is polyethylene glycol. Preferably, 1,2-epoxide polymers have amphipathicity.

As used herein, the term "polyethylene glycol (PEG)" refers to a polymer of ethylene glycol, and is also referred to as polyethylene oxide (poly(ethylene oxide)) represented by $HO-(CH_2CH_2O)_n-H$. Polyethylene glycol is commercially available from various companies, such as, for example, Union Carbide (Carbowax), Dow (Polyglycol E), Texaco Chemical (Jeffox), Olin (PolyG), BASF Wyandotte (Pluracol E), Hodag, ICI Americas (ATEG), Nacalai tesque, NOF Corporation, and the like. Typically, PEG has an average molecular weight of between 200 and 6000. Such a PEG is, for example, polyethylene glycol $(H(OCH_2CH_2)_nOH)$ having a # molecular weight of, for example, 200, 600, 1000, 2000 or 6000 from Nacalai tesque. Preferably, PEG has an average molecular weight of between 1000 and 2000. In another preferred embodiment, PEG may have an average molecular weight of 1000 or less. More preferably, PEG may have an average molecular weight of between 1500 and 2000. The above-described PEGs are commercially available and can be appropriately synthesized in order to achieve the desired characteristics. Such a synthesis method is well known in the art. Note that average molecular weights and molecular weights are herein represented by Daltons. Preferably, PEG used in the present invention has a uniform molecular weight. This is not essential. PEG having an average molecular weight within a certain range can be usually used. In the present invention, a substance (e.g., an enzyme (e.g., DNaseI or the like), an enzyme inhibitor, or the like) can be added in addition to PEG for the purpose of decellularization. When a chemical substance is added in addition to PEG, the amount or concentration of PEG to be added may vary depending on the amount of the existing chemical substance. A concentration used for cell fusion or the like is preferable. For example, a concentration of 1 g/ml or more (100 w/w or more) is preferable, but the present invention is not limited to this.

As used herein, the term "immerse" refers to placing a certain object in a certain fluid (e.g., liquid). In the present invention, the term "immerse" indicates that tissue to be treated is placed in a solution containing a non-micellar amphipathic molecule (e.g., a 1,2-epoxide polymer, such as polyethylene glycol) of the present invention for treatment. Therefore, the immersing step is preferably carried out, preferably, in a manner that permits tissue to be treated to be completely immersed in a solution for treatment. Also, in the immersing step, tissue to be treated may be preferably subjected to physical treatment (e.g., rubbing or pressing with a glass rod) in order to efficiently remove components to be removed.

As used herein, the term "washing" step indicates that after performing the step of immersing tissue to be treated in a solution a non-micellar amphipathic molecule (e.g., a 1,2-epoxide polymer) of the present invention, the solution is removed from the tissue. Therefore, preferably, the washing step is performed using liquid. In the present invention, since treated tissue is intended to be used in an organism, the tissue is preferably washed with physiologically acceptable liquid. In one preferred embodiment, the washing step may be performed using PBS (phosphate buffered saline). Awash solution may optionally contain other pharmaceutical agents (e.g., a protease inhibitor). The other pharmaceutical agents are preferably not toxic and are biocompatible.

As used herein, the term "chemical treatment" refers to treating (e.g., immersing) a certain object with a chemical substance in a broad sense. Note that as used herein, the term "chemical treatment" refers to steps other than the step of immersing an object in a solution containing a 1,2-epoxide polymer (e.g., polyethylene glycol) as an essential step. Therefore, in a method of the present invention, for example, when chemical treatment is performed in addition to the step of immersing an object in a solution containing polyethylene glycol having an average molecular weight of 1000 to 2000, chemical treatment includes the step of immersing the object in a solution other than the solution containing polyethylene glycol having an average molecular weight of 1000 to 2000 (e.g., a DNaseI solution, a glutaraldehyde solution, a solution containing polyethylene glycol having another average molecular weight, other 1,2-epoxide polymer solutions, and the like, but not limited to these solutions).

As used herein, the "physiologically active substance" refers to a substance which acts on cells or tissue. Physiologically active substances include cytokines and growth factors. Physiologically active substances may be naturally occurring or synthetic. Preferably, a physiologically active substance is one that is produced by cells or one having an action similar to that. As used herein, physiologically active substances may be in the form of a protein or nucleic acid or in other forms. When cytokines actually exert an action, the cytokines are usually in the form of proteins.

As used herein, the term "cytokine" is used in its broadest sense in the art, referring to a physiologically active substance which is produced by a cell and acts on the same or different cell. Cytokines are generally proteins or polypeptides, which have an action of controlling an immune response, an action of regulating an endocrine system, an action of regulating a nerve system, an anti-tumor action, an anti-virus action, an action of regulating proliferation, an action of regulating differentiation, and the like. As used herein, cytokines may be in the form of proteins or nucleic acids, or in other forms. When cytokines actually exert an action, the cytokines are usually in the form of proteins.

As used herein, the term "growth factor" or "cell growth factor" are used interchangeably, referring to a substance capable of promoting or controlling cell growth. Growth factors are also referred to as proliferation factors or development factors. Growth factors may be added to a medium in cell or tissue culture and may substitute for actions of serum macromolecule substances. It has been revealed that a number of growth factors can function as factors controlling differentiation of cells in addition to cell growth.

Cytokines include, typically, interleukins, chemokines, hemopoietic factors (e.g., colony stimulating factors), tumor necrosis factors, and interferons. Growth factorsinclude,typically,a platelet-derived growthfactor (PDGF), an epithelial growth factor (EGF), a fibroblast growth factor (FGF), a hepatocyte growth factor (HGF), and a vessel endothelial growth factor (VEGF), which have growth activity.

Physiologically active substances, such as cytokines and growth factors, generally have a redundancy of functions. Therefore, even a cytokines or growth factor known as having a different name and function may be used in the present invention as long as it has the same function as that of a physiologically active substance of the present invention. If cytokines or growth factors have preferable activity as set forth herein, these substances can be used in a treatment method or medicament according to a preferred embodiment of the present invention.

As used herein, the term "differentiation" refers to a developmental process of a portion of an organism, such as a cell, tissue, or an organ, in which a characteristic tissue or organ is formed. The term "differentiation" is used mainly in embryology, developmental biology, and the like. An organism forms various tissue and organs from the division of a fertilized ovum to the formation of an adult. In an early stage of development of an organismbefore division or in inadequate division, individual cells or cell groups have no morphological or functional feature and it is difficult to separate the cells or cell groups. This state is called "undifferentiated". "Differentiation" also takes place at the organ level. Cells constituting an organ develop various characteristic cells or cell groups. This is called "differentiation" in an organ during the formation of an organ. Therefore, regarding regeneration in the present invention, cell differentiation means that a cell acquires a certain morphological or functional feature which had not been possessed by the cell before treatment. For example, in the case of a cardiac valve, when a stem cell (e.g., an embryonic stem cell or a tissue stem cell) is provided, the stem cell is differentiated into a cell which is morphologically or functionally similar to a cell or tissue existing in the cardiac valve to some degree.

As used herein, the terms "graft" and "tissue graft" are used interchangeably, referring to homologous or heterologous tissue or cell group which is inserted into a particular site of a body and thereafter forms a portion of the body. Examples of grafts include, but are not limited to, organs or portions of organs, blood vessels, blood vessel-like tissue, skin segments, cardiac valves, pericardia, dura mater, corneas, bone segments, teeth, and the like. Therefore, grafts encompass any one of these which is inserted into a deficient portion so as to compensate for the deficiency. Grafts include, but are not limited to, autografts, allografts, and xenografts, which depend on the type of their donor.

As used herein, the term "autograft" refers to a graft which is implanted into the same individual from which the graft is derived. As used herein, the term "autograft" may encompass a graft from a genetically identical individual (e.g. an identical twin) in a broad sense.

As used herein, the term "allograft" refers to a graft which is implanted into an individual which is the same species but is genetically different from that from which the graft is derived. Since an allograft is genetically different from an individual (recipient) to which the graft is implanted, the graft may elicit an immune reaction. Such a graft includes, but is not limited to, for example, a graft derived from a parent.

As used herein, the term "xenograft" refers to a graft which is implanted from a different species. Therefore, for example, when a human is a recipient, a porcine-derived graft is called a xenograft.

As used herein, "recipient" (acceptor) refers to an individual which receives a graft or implanted matter and is also called "host". In contrast, an individual providing a graft or implanted matter is called "donor" (provider).

With the decellularization technique of the present invention, any graft can be used. This is because a graft (e.g., tissue, an organ, or the like) decellularized by a method of the present invention retains such a tissue damage rate that does not hinder therapy (i.e., a low tissue damage rate) and adverse effects, such as elicitation of an immune reaction, calcification, and the like, are significantly suppressed. Therefore, even in conventional situations where only an autograft is available, allografts or xenografts can be used. This is one of the significant effects of the present invention which cannot be achieved by conventional techniques.

As used herein, the term "subject" refers to an organism to which treatment of the present invention is applied and is also referred to as "patient". A patient or subject may be preferably a human.

Cells optionally used in the method or tissue graft of the present invention may be derived from a donor genetically identical to the recipient (autologous cells); a donor genetically different from, though of the same species as the recipient (homologous cells); or a donor of a species different from the recipient (heterologous cells). Considering rejection reactions, autologous cells are preferable. When rejection reactions raise no problem, homologous cells may be used. Further, if cells capable of causing rejection reactions are treated to overcome the rejection reaction, such cells can be used. A method for avoiding rejection reactions is known in the art and is described in, for example, "Shin-Geka-Taikei, Shinzo-Ishoku Hai-Ishoku Gijyutsuteki, Rinriteki-Seibi-kara-Jissi-nimukete [Heart Transplant · Lung Transplant - From Technical and Ethical Development to Practice]" (3rd Version). Examples of such a method include use of an immunosuppressant or a steroid drug, and the like. Immunosuppressants for preventing rejection reactions currently include "cyclosporine" (SANDIMMUNE/NEORAL), "tacrolimus" (PROGRAF), "azathioprine" (IMURAN), "steroid hormone" (prednine, methylprednine), and "T-cell antibodies" (OKT3, ATG, etc.). A method which is used for preventive immunosuppressive therapy in a number of facilities in the world is three-drug therapy using "cyclosporine, azathioprine, and steroid hormone". An immunosuppressant is preferably, but not necessarily, administered concomitantly with a medicament of the present invention. Therefore, an immunosuppressant may be administered before or after a regeneration/therapeutic method of the present invention as long as an immunosuppression effect can be achieved.

Cells used in the present invention may be derived from any organism (e.g., vertebrates and invertebrates). Preferably, cells derived from vertebrates are used. More preferably, cells derived from mammals (e.g., primates, rodents, etc.) are used. Even more preferably, cells derived from primates are used. Most preferably, cells derived from humans are used.

Combinations of a subject targeted by the present invention and decellularized tissue include, but not limited to, for example, implantation to the heart suffering from a heart disease (e.g., ischemic heart diseases); pericardia patch, implantation of dura mater in brain surgery; implantation of a blood vessel to treat cardiac infarction, a lower limb, an upper limb, or the like; implantation of a bone to a patient suffering from bone fracture or bone failure; implantation of cornea of the present invention to a patient having an injured cornea; and the like.

Tissue targeted by the present invention may be any organ or part of an organism or may be derived from any kind of organism. Organisms targeted by the present invention include vertebrates or invertebrates. Preferably, organisms targeted by the present invention are mammals (e.g., primates, rodents, etc.). More preferably, organisms targeted by the present invention are primates. Most preferably, organisms targeted by the present invention are humans.

Attention has been attracted by blood vessel regeneration therapy using self cells, and a method for implanting tissue can be carried out as well known in the art. In the cardiovascular surgery region, various prosthetic valves or vascular prostheses or the like are used, however, a number of problems arise, such as their durability or necessity of anticoagulant therapy, and associated hemorrhagic tendency and susceptibility to infection, and the like. Therefore, there is an expectation for regeneration medical engineering. Niioka et al. prepared regenerated blood vessels by culturing blood vessel smooth muscle cells from a vein of a foot of a 4-year old female with pulmonary artery deficiency on a bioabsorbable high molecular weight polymer and carried out implantation of the regenerated blood vessel (Shunji Niioka, Yasuharu Imai, Kazuhiro Seo, et al.; "Tissu-Enjinearinngu-niyoru-Shinkekkanzairyo-no-Kaihats u, Oyo [Development and Application of Cardiovascular Material in Tissue Engineering]", Nichi Shinzo Kekkan Gekaishi, 2000; 29: 38). In tissue engineering in the cardiovascular system, implanted cells or structures can make direct contact with blood in a blood vessel and are therefore supplied with oxygen and nutrients immediately after implantation. Thus, implanted cells or structures are considered to be under advantageous conditions. Particularly, use of self cells (cell replacement) has various advantages as follows, for example.

1. Removal of the possibility of a rejection reaction.
2. No necessity of considering a donor.
3. Expectation of long durability due to utilization of living tissue.
4. No residual foreign matter since a scaffold polymer is completely biodegraded when cells complete the extracellular stroma.
5. Excellent antithrombogenicity due to eventual coverage with endothelia and no necessity of anticoagulant therapy after implantation.
6. Expectation of growth due to autologous tissue.

At present, self cells are used in the right arterial system within a blood pres sure range around a pulmonary artery pressure. However, self cells can be appropriately applied to use with an aorta pressure, artery grafts for valve tissue or AC bypass, tendinous cord tissue, or the like ("Junkanki-Shikkan-no-Saishin-Tiryo 2002-2003 [Up-to-date Therapy for Cardiovascular diseases 2002-2003]"; Nankodo, p. 29, published in 2002).

General.use of prosthetic valves is well known in the art. The present invention is also carried out based on this well-known matter. For example, stentless heterologous tissue valves are known. In heterologous tissue valves, the presence of a stent reduces the effective area of the valve port, leading to calcification or degeneration of valve leaflets. Recently, by utilizing the morphology of the base portion of the porcine aorta, a stentless heterologous tissue valve stent without a stent has attracted attention as a prosthetic valve for the aortic valve (Gross C., et al., Ann. Thorac. Surg., 68: 919, 1999). It is considered that the absence of a stent results in a small pressure difference across the valve even when a small-size valve is unavoidably used and is also effective for postoperative enlargement of the left ventricle. Further, the elasticity of the base portion of the aorta is maintained, stress to the cusp is small, and the durability can be expected to be improved as compared to a tissue valve with a stent. Further, stentless heterologous tissue valves can be used in the case of endocarditis due to infection or prosthetic valve infection. At present, substantially satisfactory intermediate-term postoperative results of stentless heterologous tissue valves have been reported in the USA and Europe, and long-term results can be expected to be satisfactory (Gross C., et al., Ann. Thorac. Surg., 68:919, 1999).

BEST MODE FOR CARRYING OUT THE INVENTION

According to one aspect of the present invention, decellularized tissue is provided. The decellularized tissue of the present invention has the following features: A) the cell survival rate of the tissue is less than or equal to a level at which an immune reaction is elicited in an organism; and B) the tissue is not damaged to such an extent that hinders the tissue from exhibiting a function which was possessed by the tissue when the tissue was not treated. Cells remaining in tissue cause calcification. Moreover, when non-autologous tissue is implanted, the residual cells are likely to elicit an undesired immune reaction. Therefore, the decellularized tissue of the present invention has to have such small a number of cells as that it does not elicit an immune reaction in organisms. Thus, cells are preferably removed as much as possible. In order that the decellularized tissue of the present invention is used in implantation therapy, a tissue or an organ must not be damaged to such an extent that the tissue is hindered from exhibiting a function which was possessed by the tissue before treatment (decellularization). The presence or absence of such hinderance can be determined by confirming that the extracellular matrix of the tissue is not substantially degenerated. Therefore, the tissue of the present invention may be characterized in that the extracellular matrix substantially remains. The presence of extracellular matrices can be confirmed by staining using a marker specific thereto. As described above, the tissue of the present invention has excellent physical strength, pressure-resistant property, and the like and therefore is preferably used as a scaffold for recellularization (or cell replacement) in a recipient. Conventionally, there is a method for reducing a cell survival rate in tissue (Koide A., Hayashi T., eds (2000); "Basic and Clinical Studies of Extracellular Matrix (ECM); Aichi Shuppan Co., Ltd., Tokyo, Japan CAN: 133.333569). However, when a conventional method is used to reduce a cell survival rate to 30% or less, tissue is damaged to such an extent that the tissue cannot withstand organ implantation. The present invention achieves a significant effect in that cell survival rate can be reduced while tissue or an organ is not damaged to such an extent that hinders the tissue or organ from exhibiting a normal function which was possessed by the tissue or organ before treatment (decellularization). The present invention can be used with cells or without a cell.

In order to present the above-described adverse effects, the cell survival rate of the decellularized tissue according to one embodiment of the present invention is typically about 50% or less, representatively about 40% or less, preferably about 30% or less, more preferably about 25% or less, still more preferably about 20% or less, still even more preferably about 15% or less, still even more preferably about 10% or less, and most preferably about 5% or less. The decellularized tissue of the present invention is characterized in that although the cell survival rate thereof is as low as such a degree, the tissue damage rate thereof is as low as a level that permits use in clinical applications. Unless a function originally possessed by tissue (before treatment) can be exhibited, the tissue cannot be used in clinical applications no matter how low the cell survival rate is. Therefore, as the tissue damage rate of tissue is decreased while keeping the above-described cell survival rate, the tissue is more preferable. Inotherwords, tissue has to maintain a low tissue damage rate which permits clinical applications.

The smaller the tissue damage rate which permits tissue to be used in clinical applications, the more preferable the tissue of the present invention. The tissue damage rate may be typically about 50% or less, representatively about 40% or less, preferably about 30% or less, more preferably about 25% or less, still more preferably about 20% or less, still even more preferably about 15% or less, still even more preferably about 10% or less, and most preferably about 5% or less. Even though the tissue damage rate is decreased in this manner, it is not possible to use tissue or an organ which is damaged to such an extent that the tissue or organ cannot exhibit an originally possessed function thereof. Therefore, in order to achieve an objective of the present invention, it is preferable that the tissue of the present invention is not damaged to such an extent that the tissue is hindered from exhibiting an originally possessed function thereof. It is possible to evaluate the tissue damage rate to determine whether or not the tissue can exhibit an originally possessed function thereof. The tissue damage rate can be evaluated by the above-described method. Alternatively, the decellularized tissue of the present invention can be determined by evaluating the survival rate of extracellular matrices. The survival rate of extracellular matrices may be, for example, about 50% or more, preferably about 70% or more, more preferably about 80% or more, and even more preferably about 90% or more.

Therefore, in a preferred embodiment, the decellularized tissue has 1) a cell survival rate of about 30% or less; and 2) a tissue damage rate of about 30% or less. In a more preferred embodiment, the decellularized tissue has 1) a cell survival rate of about 15% or less; and 2) a tissue damage rate of about 15% or less. In a still more preferred embodiment, the decellularized tissue has 1) a cell survival rate of about 5% or less; and 2) a tissue damage rate of about 15% or less. These embodiments are provided only for illustrative purposes and are not intended to limit the scope of the present invention.

In one embodiment, the decellularized tissue of the present invention has such a tissue strength that permits clinical applications. A sufficiently high level of tissue strength is an important characteristic, particularly when membrane-like tissue is used in clinical applications. Tissue strength can be generally determined by measuring tensile strength (e.g., breaking strength, stiffness, Young's modulus, etc.). In a certain embodiment, the decellularized tissue of the present invention may have a tissue strength which is at least about 75% or more of the strength which was possessed by the tissue before treatment, preferably about 80% or more, more preferably about 85% or more, still more preferably about 90% or more, or alternatively may have a tissue strength greater than or equal to that which was possessed by the untreated tissue (originally possessed tissue strength). As usedherein, the term "tissue strength of untreated tissue" refers to tissue strength which was possessed by tissue before treatment (e.g., treatment with a 1,2-epoxide polymer of the present invention). For example, untreated tissue is naturally occurring. Sufficiently strong tissue strength is a preferable characteristic even when a certain kind of tissue (e.g., lumenal tissue) is used other than membrane-like tissue. In the case of lumenal tissue, tissue strength can be represented by a $\beta$ value. A method for calculating a $\beta$ value is heretofore described in detail and is also illustrated in examples below. In a certain embodiment, the decellularized tissue of the present invention has a tissue strength having a $\beta$ value of about 15 or more, preferably a $\beta$ value of about 18 or more, more preferably a $\beta$ value of about 20 or more, and still more preferably a $\beta$ value of about 22 or more. In another embodiment, the decellularized tissue of the present invention may have a $\beta$ value which is at least about 75% or more of that which was possessed by the tissue before treatment, preferably about 80% or more, more preferably about 85% or more, still more preferably about 90% or more, or alternatively may have a $\beta$ value greater than or equal to that which was possessed by the untreated tissue (originally possessed $\beta$ value). As used herein, the term "characteristic of untreated tissue" refers to a characteristic which was possessed by tissue before treatment (e.g., treatment with a 1,2-epoxide polymer of the present invention). For example, untreated tissue is naturally occurring. Therefore, for example, when original tissue had a $\beta$ value of 25, the decellularized tissue of the present invention may preferably have a $\beta$ value of 17.5 or more, more preferably 20 or more, still more preferably 21.25 or more, and still even more preferably 22.5 or more.

The decellularized tissue of the present invention may be tissue which is derived from any part of the body if the tissue is intended to be used in clinical applications. In a certain embodiment, the decellularized tissue of the present invention may require physical structure. In order to maintain physical structure, only structure, such as cytoskeleton and the like, is required, while intracellular components, such as plasma components and the like, are not necessarily required. Also, optionally required cells may be additionally provided to decellularized tissue or may be internally supplied from a host to which the tissue is implanted. In a certain embodiment, the decellularized tissue of the present invention may be derived from an organ selected from blood vessels, blood vessel-like tissue, cardiac valves, pericardia, dura mater, corneas, and bones. In another embodiment, the decellularized tissue of the present invention may be derived from an organ selected from cardiovascular tissue (e.g., blood vessels, blood vessel-like tissue, cardiac valves, and pericardia).

The decellularized tissue of the present invention may be tissue derived from any organism if the tissue is suitable for an intended clinical application. Therefore, the tissue of the present invention may be derived from any organism (e.g., vertebrates and invertebrates). When intended to be applied to a human, vertebrate-derived tissue is preferably used, and more preferably tissue derived from a mammal (e.g., a primate, a rodent, or the like) is used. When intended to be applied to a human, primate-derived tissue is more preferably used. When intended to be applied to a human, porcine-derived tissue is more preferably used. This is because porcine-derived tissue has a size similar to that of a human. When intended to be applied to a human, human-derived tissue is most preferably used. The decellularized tissue or tissue graft of the present invention preferably has a size similar to that of a human and a physical characteristic similar to that of a human, when the tissue or tissue graft is derived from organisms other than a human (e.g., swine).

In the present invention, the decellularized tissue of the present invention may be applied to any part of the body of an organism. Therefore, the decellularized tissue may be applied to a part of the body from which the decellularized tissue was derived or other parts of the body. As shown in the Examples, it was demonstrated that the desired effect (e.g., regeneration, self organization) of the present invention can be achieved for any part of the body to which the decellularized tissue of the present invention is applied, no matter whether or not the part is a site from which the tissue was derived. Therefore, the present invention has a considerably advantageous utility such that the present invention can in principle be applied to any implantation operation and regeneration operation. Examples of parts to which the decellularized tissue of the present invention is applied, include, but are not limited to, skin, blood vessel, cornea, kidney, heart, liver, umbilical cord, intestine, nerve, lung, placenta, pancreas, brain, peripheral limb, retina, valve, epithelial tissue, connective tissue, muscluar tissue, nervous tissue, and the like.

In another aspect, the present invention provides a tissue graft containing the decellularized tissue of the present invention. In this tissue graft, recipient-derived cells are disseminated and cultured in the above-described decellularized tissue so that desired tissue structure is formed. The tissue graft of the present invention may be intended to be implanted into any tissue in the body as long as the tissue graft is of tissue intended for a clinical application. In a certain embodiment, the tissue graft of the present invention may require physical structure. In order to maintain physical structure, recipient-derived cells may be provided to the above-described decellularized tissue before implantation. The recipient-derived cells may be internally supplied from a host to which the tissue graft is implanted. In a certain embodiment, the tis sue graft of the present invention may be derived from an organ selected from blood vessels,blood vessel-like tissue, cardiacvalves, pericardia, dura mater, corneas, and bones. In another embodiment, the tissue graft of the present invention may be derived from an organ selected from cardiovascular tissue (e.g., blood vessels, blood vessel-like tissue, cardiac valves, and pericardia).

The tissue graft of the present invention may contain tissue derived from any organism if the tissue is suitable for an intended clinical application. Therefore, the tissue graft of the present invention may contain tissue derived from any organism (e.g., vertebrates and invertebrates). When intended to be applied to a human, vertebrate-derived tissue is preferably used for the tissue graft of the present invention, and more preferably tissue derived from a mammal (e.g., a primate, a rodent, or the like) is used for the tissue graft of the present invention. When intended to be applied to a human, primate-derived tissue is more preferably used for the tissue graft of the present invention. When intended to be applied to a human, porcine-derived tissue is more preferably used for the tissue graft of the present invention. This is because porcine-derived tissue has a size similar to that of a human. When intended to be applied to a human, human-derived tissue is most preferably used.

Recipient-derived cells used in the tissue graft of the present invention may be any cells that are suitable for clinical applications. Therefore, the cell may be selected from the group consisting of vascular endothelial cells, smooth muscle cells, fibroblasts, blood cells, and precursor cells and somatic stem cells capable of differentiating into those cells. Preferably, the cell may be a cell capable of exhibiting a desired function at a site at which the cell is implanted.

In the present invention, the tissue graft of the present invention may be applied to any part of the body of an organism. Therefore, the tissue graft may be applied to a part of the body from which the tissue graft was derived or other parts of the body. As shown in the Examples, it was demonstrated that the desired effect (e.g., regeneration, self organization) of the present invention can be achieved for any part of the body to which the tissue graft of the present invention is applied, no matter whether or not the part is a site from which the tissue was derived. Therefore, the present invention has a considerably advantageous utility such that the present invention can in principle be applied to any implantation operation and regeneration operation. Examples of parts to which the tissue graft of the present invention is applied, include, but are not limited to, skin, blood vessel, cornea, kidney, heart, liver, umbilical cord, intestine, nerve, lung, placenta, pancreas, brain, peripheral limb, retina, valve, epithelial tissue, connective tissue, muscluar tissue, nervous tissue, and the like.

In another aspect, the present invention provides a membrane-like tissue graft. This membrane-like tissue graft comprises A) the decellularized tissue of the present invention. Here, recipient-derived cells are disseminated and cultured in the decellularized tissue so that desired tissue structure is formed.

In another aspect, the present invention provides a method of producing decellularized tissue. This method comprises the steps of A) providing tissue; and B) immersing the tissue in a solution containing a non-micellar amphipathic molecule (e.g., a 1,2-epoxide polymer). Any amphipathic molecule which is not micellar can be used. As the amphipathic molecule, any polymer which can remove plasma components can be used. The non-micellar amphipathic molecule used in the present invention is preferably a 1,2-epoxidepolymer,and more preferably polyethylene glycol (PEG), though any non-micellar amphipathic molecule can be used. Amphipathic molecules, conventionally called surfactants, can be used. In these situations, it is preferable to confirm that an amphipathic molecule is not micellar before using a solution containing the amphipathic molecule. The confirmation of the non-micellar state can be performed by using a well-known technique in the art. For example, such a technique includes, but is not limited to, visual inspection, absorbance measurement, and the like.

In one preferred embodiment, the average molecular weight of PEG is between 200 and 6000. PEG used in the present invention may have various average molecular weights. By changing treatment time (immersion time) depending on the average molecular weight, a desired effect (e.g., decellularization) can be achieved. In one preferred embodiment, the average molecular weight of PEG used in the present invention is between 1000 and 2000. In another preferred embodiment, the average molecular weight of PEG used in the present invention is between 1500 and 2000. In another embodiment, the average molecular weight of PEG used in the present invention is smaller than or equal to 1000. In general, as the average molecular weight of PEG used is increased, the treatment time has to be decreased. This is because as the average molecular weight of PEG used is increased, the effect of extracting intracellular components increases. Therefore, in one embodiment, tissue may be immersed in a 1,2-epoxide polymer-containing solution for 30 min to 60 min. A minimum time required in the immersing step varies depending on the amphipathic molecule used (e.g., a 1,2-epoxide polymer, such as PEG), but can be determined by those skilled in the art without undue experimentation. Therefore, the step of immersing tissue in a solution containing an amphipathic molecule, such as a 1,2-epoxide polymer, may be performed for less than 30 min. Alternatively, the immersing step using a solution containing an amphipathic molecule, such as a 1,2-epoxide polymer, may be performed for more than 60 min. A minimum time can be obtained as follows: various treatment times are provided in control experiments; at each time point, the state of treated tissue is determined by measuring the tissue damage rate, the cell survival rate, or the like of the tissue by methods as described herein; and based on the result, it is determined whether or not the treated tissue has an appropriate characteristic. In the method of the present invention, the step of immersing tissue in the solution may be performed under any condition and may be performed typically at a temperature of between 0°C and 42°C, at room temperature (between about 15°C and about 25°C), or at 37°C. This step may be performed at a temperature of more than 37°C. In the method of the present invention, the 1,2-epoxide polymer (e.g., PEG) may be present in the solution at any concentration and may be preferably present at a concentration of 1 g/ml or more. Note that when PEG (molecular weight: 1000) is used as a material reagent, it is solid at room temperature and therefore may be dissolved in water. In the method of the present invention, the 1,2-epoxide polymer (e.g., PEG) may be dissolved in any solvent and may be preferably dissolved in an aqueous medium, more preferably physiological saline, PBS (phosphate buffered saline), or other solutions containing salts. Such a solution may be any solution in which an amphipathic molecule used in the present invention is not micellar at a concentration which is usually used for the solution. The solution having a non-micellar amphipathic molecule (e.g., a 1,2-epoxide polymer) is preferably sterilized. A non-micellar amphipathic molecule (e.g., a 1,2-epoxide polymer) used in the present invention is preferably biocompatible. Such a biocompatible, non-micellar amphipathic molecule (e.g., a 1,2-epoxide polymer) includes, but is not limited to, polymers which are biocompatible and are of the medicament grade, for example, PEG, segmented polyurethane, silicone, MMA (α-methylmethacrylate (contact lens material), etc.), and PTFE (polytetrafluoroethylene) (e.g., Teflon (registered trademark), Dacron, etc.). Such a biocompatible 1,2-epoxide polymer is described in, for example, Japanese Pharmacopoeia (or counterparts in other countries) or may be a polymer approved by the Health, Labor and Welfare Ministry (or counterparts in other countries).

Preferably, in the method of the present invention, the step of immersing tissue in a solution containing a 1, 2-epoxide polymer may be performed concomitantly or before treatment with DNase (e.g., DNaseI).

Preferably, the method of the present invention further comprises washing the tissue immersed in the solution. The washing step can be performed using any liquid which is physiologically suitable (e.g., physiological saline, PBS (phosphate buffered saline), etc.). In a preferred embodiment, the washing step in the present invention is performed with PBS. The wash solution is preferably sterilized. The washing step may be performed under any condition and may be performed typically at a temperature of between 0°C and 42°C, at room temperature (about 15°C and about 25°C), or at 37°C. The washing step may be performed at a temperature of more than 37°C. In the method of the present invention, the washing step may be performed for any period of time as long as the solution containing an amphipathic molecule, such as a 1,2-epoxide polymer, can be sufficiently removed and may be usually performed for 3 days to 5 days. Preferably, in the washing step, a wash solution (e.g., PBS) may be changed several times.

Tissue used in the method of the present invention may be tissue derived from any organism if the tissue is suitable for an intended clinical application. Therefore, tissue used in the method of the present invention may be derived from any organism (e.g., vertebrates and invertebrates). When intended to be applied to a human, vertebrate-derived tissue is preferably used as tissue used in the method of the present invention, and more preferably tissue derived from a mammal (e.g., a primate, a rodent, or the like) is used as tissue used in the method of the present invention. When intended to be applied to a human, primate-derived tissue is more preferably used as tissue used in the method of the present invention. When intended to be applied to a human, porcine-derived tissue is more preferably used. This is because porcine-derived tissue has a size similar to that of a human. When intended to be applied to a human, human-derived tissue is most preferably used.

Tissue used in the method of the present invention may be any tissue in the body as long as the tissue is intended to be used in clinical applications. In a certain embodiment, the tissue used in the method of the present invention may require physical structure or physical properties. In order to maintain physical structure or physical properties, only structure, such as extracellular matrices and the like, are required, while intracellular components, such as plasma components, cell membrane components, and the like, are not necessarily required. Also, optionally required cells may be additionally provided to decellularized tissue or may be internally supplied from a host to which the tissue is implanted. In a certain embodiment, tissue used in the method of the present invention may be derived from an organ selected from blood vessels, blood vessel-like tissue, cardiacvalves, pericardia, dura mater, corneas, and bones. In another embodiment, the decellularized tissue of the present invention may be derived from an organ selected from cardiovascular tissue (e.g., blood vessels, blood vessel-like tissue, cardiac valves, and pericardia).

In a preferred embodiment, the method of the present inventionmay further comprise performing chemical treatment. Here, the chemical treatment may be one other than the step of immersing tissue in a solution containing a non-micellar amphipathic molecule (e.g., a 1,2-epoxide polymer, such as polyethylene glycol) in the method of the present invention. Alternatively, the chemical treatment may be the same as the step of immersing tissue in a solution containing a non-micellar amphipathic molecule (e.g., a 1,2-epoxide polymer, such as polyethylene glycol) in the method of the present invention (i.e., repeating the immersing step). Therefore, in the method of the present invention, for example, when the chemical treatment is performed in addition to the step of immersing tissue in a solution containing polyethylene glycol having an average molecular weight of 1000 to 2000, the chemical treatment comprises the step of immersing tissue in a solution other than the solution containing polyethylene glycol having an average molecular weight of 1000 to 2000 (including, but not limited to, for example, a solution containing DNase (e.g., DNaseI), a glutaraldehyde-containing solution, a solution containing polyethylene glycol having another average molecular weight, a solution containing another non-micellar amphipathic molecule (e.g., a 1,2-epoxide polymer) in the present invention, and the like). Alternatively, for example, when the chemical treatment is performed in addition to the step of immersing tissue in the solution containing polyethylene glycol having an average molecular weight of 1000 to 2000, the chemical treatment may comprise repeating the step of immersing tissue in the solution containing polyethylene glycol having an average molecular weight of 1000 to 2000.

In one embodiment, the above-described chemical treatment may be treatment with a difunctional molecular cross-linking agent (e.g., glutaraldehyde or a derivative thereof). The treatment with a difunctional molecular cross-linking agent is intended to chemically cross-link a protein component contained in ECM or tissue cells to increase physical strength. Therefore, if this purpose can be achieved, any difunctional molecular cross-linking agent can be used. Among such difunctional molecular cross-linking agents, some agents which can be actually used for fixation of tissue (valve graft) include, but are not limited to, for example, cyanimide, 1-ethyl-3-(3-dimethylaminopropyl)carbodiimidehydrochlor ide (EDC), epoxy(poly(glycidylether)) or photocross-linking agent PhotoMix™ (Sulzer Carbomedics Co., Ltd.) (see, Biomaterials (2000) 21:2215-2231). In another embodiment, the chemical treatment may comprise treatment with DNase, such as DNaseI. DNA components undesirable for implantation can be removed by means of the treatment with DNase. Therefore, the treatment with DNase is preferable. Such DNase may be any DNase and may be preferably DNaseI. With the DNase treatment, DNA, which is a charged polymer substance, can be removed. DNA has the possibility of eliciting an immune reaction. Therefore, by removing DNA, an advantage can be provided.

The present invention also provides decellularized tissue obtained by the decellularization method of the present invention. This decellularized tissue may pref erably have the above-described cell survival rate and/or tissue damage rate and/or tissue strength. Before the decellularization method of the present invention is provided, it was not possible to provide decellularized tissue having the above-described cell survival rate and/or tissue damage rate and/or tissue strength. Thus, the decellularization method of the present invention has an unexpected advantage of providing decellularized tissue having a characteristic which could not be provided by conventional methods.

In another aspect, the present invention provides a method of regenerating tissue. This method comprises the steps of A) providing the decellularized tissue of the present invention; B) providing a physiologically active substance capable of inducing differentiation of cells in the tissue; and C) incubating the cells for a sufficient time for differentiation. The method may optionally comprise providing cells to the decellularized tissue or the decellularized tissue may not be provided with a cell. Preferably, the cell may be a blood vessel cell or a blood vessel-like cell. More preferably, the cell may be derived from a recipient. Preferably, the tissue may be selected from the group consisting of bloodvessels, blood vessel-like tissue, cardiac valves, pericardia, dura mater, corneas, and bones. In a preferred embodiment, the tissue and the cell may be derived from the same host. In another embodiment, the tissue and the cell may be derived from homologous hosts. In another embodiment, the tissue and the cell may be derived from heterologous hosts. When the recipient and the cell are homologous or heterologous, a rejection reaction is expected. Therefore, the recipient and the cell are preferably derived from the same host. When a rejection reaction does not raise a problem, the recipient and the cell may be homologous or heterologous. If the cell which elicits a rejection reaction is optionally treated to overcome the rejection reaction, the cell can be used. A method for overcoming a rejection reaction is known in the art and is described in, for example, "Shin-Geka-Taikei, Shinzo-Ishoku Hai-Ishoku Gijyutsuteki, Rinriteki-Seibi-kara-Jissi-nimukete [Heart Transplant Lung Transplant - From Technical and Ethical Development to Practice]" (3rd Version). Such a method includes, for example, use of an immunosuppressant or a steroid, and the like. Immunosuppressants for preventing rejection reactions currently include "cyclosporine" (SANDIMMUNE/NEORAL), "tacrolimus" (PROGRAF), "azathioprine" (IMURAN), "steroid hormone" (prednine, methylprednine), "T-cell antibodies" (OKT3, ATG, etc.). A method which is used for preventive immunosuppressive therapy in a number of facilities in the world is three-drug therapy using "cyclosporine, azathioprine, and steroid hormone". An immunosuppressant is preferably, but not necessarily, administered concomitantly with a medicament of the present invention. Therefore, an immunosuppressant may be administered before or after a regeneration method of the present invention as long as an immunosuppression effect can be achieved.

In another aspect, the present invention provides a method of producing a tissue graft. This method comprises the steps of A) providing the decellularized tissue of the present invention into an organism; B) allowing self cells in the organism to infiltrate the decellularized tissue; and C) incubating the tissue for a sufficient time to differentiate the self cells. In this case, the decellularized tissue of the present invention may or may not have additional cells. The additional cells may be autologous, homologous, or heterologous. Preferably, the cell may be a blood vessel cell or a blood vessel-like cell. More preferably, the cell may be derived from a recipient. Preferably, the tissue may be selected from the group consisting of blood vessels, blood vessel-like tissue, cardiac valves, pericardia, dura mater, corneas, and bones. In a preferred embodiment, the tissue and the cell may be derived from the same host. In another embodiment, the tissue and the cell may be derived from homologous hosts. In another embodiment, the tissue and the cell may be derived from heterologous hosts. When the recipient and the cell are homologous or heterologous, a rejection reaction is expected. Therefore, the recipient and the cell are preferably derived from the same host. When a rejection reaction does not raise a problem, the recipient and the cell may be homologous or heterologous. If the cell which elicits a rejection reaction is optionally treated to overcome the rejection reaction, the cell can be used. A method for overcoming a rejection reaction is herein described.

In a preferred embodiment, the method of the tissue graft of the present invention may further comprise D) providing a physiologically active substance capable of inducing differentiation of the above-described cell. Preferably, the physiologically active substance may be a cytokine having hematopoiesis activity. In order to produce a tissue graft, the cytokine may be HGF, VEGF, FGF, or the like. The cell may be either autologous or heterologous.

In another aspect, the present invention provides a tissue graft produced by the method of the present invention. Such a tissue graft has a characteristic which is not conventionally obtained with respect to the tissue strength and the decellularization rate.

In another aspect, the present invention provides a method for treating a subject requiring implantation of tissue or an organ or treating a subject at a risk of implantation of tissue or an organ for prophylaxis. This method comprises A) providing the decellularized tissue or tissue graft of the present invention; and B) implanting the decellularized tissue to the subject. The decellularized tissue optionally contains additional cells. The cell may be autologous or homologous. Preferably, the tissue may be selected from the group consisting of blood vessels, blood vessel-like tissues, cardiac valves, pericardia, dura mater, corneas, and bones. In a preferred embodiment, the tissue may be derived from the subject. In another embodiment, the tissue may be derived from a host homologous to the subject. In another embodiment, the tissue may be derived from a host heterologous to the subject. The therapeutic/prophylactic method of the present invention employs tissue which is sufficiently decellularized that a tissue damage rate is reduced to such a level that permits implantation. Therefore, the tissue does not elicit a rejection reaction. In the case that a rejection reaction is elicited by the tissue or non-recipient-derived cells, treatment for overcoming a rejection reaction can be optionally performed. A method for overcoming a rejection reaction is herein described in detail. In one embodiment, tissue used in the treatment or prophylactic method of the present invention maybe derived from the subject. In another embodiment, tissue used in the treatment or prophylactic method of the present invention may be derived from any organism (e.g., vertebrates and invertebrates). Preferably, when a human is treated for therapy or prophylaxis, vertebrate-derived tissue may be used. More preferably, when a human is treated for therapy or prophylaxis, tissue derived from a mammal (e.g., a primate, a rodent, etc.) may be used. Still more preferably, when a human is treated for therapy or prophylaxis, primate-derived tissue may be used. In another preferred embodiment, when a human is treated for therapy or prophylaxis, porcine-derived tissue may be used. This is because porcine-derived tissue has a size similar to that of a human. Most preferably, when a human is treated for therapy or prophylaxis, human-derived tissue may be used.

In another aspect, the present invention provides a medicament for organ implantation. This medicament comprises the decellularized tissue of the present invention or the tissue graft of the present invention. In a certain embodiment, the medicament of the present invention comprises tissue derived from an organ selected from blood vessels, blood vessel-like tissue, cardiac valves, pericardia, dura mater, corneas, and bones. In another embodiment, the medicament of the present invention may comprise cardiovascular tissue, for example, tissue derived from an organ selected from blood vessels, blood vessel-like tissue, cardiac valves, and pericardia.

The medicament of the present invention may comprise tissue derived from any organism as long as the tissue is suitable for an intended clinical application. Preferably, the medicament may comprise a material approved by an authority in a country in which the medicament is used. Therefore, the medicament of the present invention may comprise tissue derived from any organism (e.g., vertebrates and invertebrates). In the medicament of the present invention, when intended to apply the medicament to a human, preferably, vertebrate-derived tissue is used. More preferably, tissue from a mammal (e.g., a primate, a rodent, etc.) is used. In the medicament of the present invention, when intended to apply the medicament to a human, still more preferably, primate-derived tissue is used. When the medicament of the present invention is intended to be applied to a human, still even more preferably, porcine-derived tissue is used. This is because porcine-derived tissue has a size similar to that of a human. When the medicament of the present invention is intended to be applied to a human, most preferably, human-derived tissue is used. Note that when human-derived tissue is used, ethical regulations or problems have to be solved.

The medicament, tissue graft and decellularized tissue of the present invention may further comprise biocompatible material. For example, the biocompatible material may comprise at least one selected from the group consisting of silicone, collagen, gelatin, glycolic acid/lactic acid copolymers, ethylene/vinyl acetate copolymers, polyurethane, polyethylene, polytetrafluoroethylene, polypropylene, polyacrylate, and polymethacrylate. Silicone is preferable because of ease to mold. Examples of biodegradable polymers include collagen, gelatin, polymers or copolymers synthesized by non-catalytic dehydrocondensation of at least one selected from $\alpha$-hydroxycarboxylic acids (e.g., glycolic acid, lactic acid, hydroxybutyric acid, etc.), hydroxydicarboxylicacids (e.g., malic acid, etc.), and hydroxytricarboxylic acids (e.g., citric acid, etc.) or a mixture thereof, poly-$\alpha$-cyanoacrylic ester, polyamino acids (e.g., poly-$\gamma$-benzyl-L-glutamic acid, etc.), and polyanhydrides such as maleic anhydride copolymers (e.g., styrene-maleic acid copolymers, etc.), and the like. These copolymers may be any of random, block, and graft copolymers. When $\alpha$-hydroxycarboxylic acids, hydroxydicarboxylic acids, and hydroxytricarboxylic acids have an optical activity center within the molecule, the molecule can be in any of the D-form, L-form, and DL-form. In a certain embodiment, a glycolic acid/lactic acid copolymer may be used.

The medicament, tissue graft, and decellularized tissue of the present invention may further comprise another pharmaceutical agent. Such a pharmaceutical agent may include any pharmaceutical agent known in the field of pharmaceuticals. The medicament, tissue graft, and decellularized tissue of the present invention may comprise two or more other pharmaceutical agents. Such pharmaceutical agents include, for example, those which are described in the up-to-date version of Japanese Pharmacopoeia, US Pharmacopoeia, pharmacopoeias in other countries, and the like. The pharmaceutical agent may preferably have an effect on an organ of an organism. The pharmaceutical agent includes, for example, thrombolytic agents, vasodepressors, and tissue activating agents. The amounts of a physiologically active substance and other pharmaceutical agents and cells contained in the medicament, tissue graft, and decellularized tissue of the present invention can be easily determined by those skilled in the art, considering purposes of use, target diseases (type, severity, etc.), patient's age, weight, sex, case history, and the like.

In another aspect, the present invention relates to use of the decellularized tissue of the present invention or the tissue graft of the present invention for organ implantation and medicament production. In a certain embodiment, regarding the use of the present invention, tissue derived from an organ selected from blood vessels, blood vessel-like tissue, cardiac valves, pericardia, dura mater, corneas, and bones, may be used. In another embodiment, regarding the use of the present invention, tissue derived from an organ selected from cardiovascular tissue, such as blood vessels, blood vessel-like tissue, cardiac valves, and pericardia, may be used.

Regarding the use of the present invention, tissue derived from any organism may be used as long as the tissue is suitable for an intended clinical application. Preferably, a material approved by an authority in a country in which the medicament is used, may be used. Therefore, regarding the use of the present invention, tissue derived from any organism (e.g., vertebrates and invertebrates) may be used. Regarding the use of the present invention, when intended to apply the present invention to a human, preferably, vertebrate-derived tissue is used. More preferably, tissue from a mammal (e.g., a primate, a rodent, etc.) is used. Regarding the use of the present invention, when intended to apply the present invention to a human, still more preferably, primate-derived tissue is used. When the present invention is intended to be applied to a human, still even more preferably, porcine-derived tissue is used. This is because porcine-derived tissue has a size similar to that of a human. When the present invention is intended to be applied to a human, most preferably, human-derived tissue isused. Note that when human-derived tissue is used, ethical regulations or problems have to be solved.

The use of the decellularized tissue, graft, and medicament of the present invention is usually performed under supervision of a doctor, or without supervision of a doctor if approved by an authority and a law of a country in which the present invention is used.

The amounts of decellularized tissue, a graft, and a medicament used in the treatment or prophylactic method of the present invention can be easily determined by those skilled in the art, considering purposes of use, target diseases (type, severity, etc.), patient's age, weight, sex, case history, the form or type of a physiologically active substance, the form or type of the tissue, or the like.

The frequency of the method of the present invention being applied to a subject (or a patient) can be easily determined by those skilled in the art, considering the doses of decellularized tissue, a graft, and a medicament, purposes of use, targeted diseases (type, severity, etc.), patient's age, weight, sex, case history, the course of therapy, and the like. The frequency includes, for example, once per day to once per several months (e.g., once per week to once per month). Preferably, administration is carried out once per week to once per month while observing progress.

As used herein, molecular biological techniques, biochemical techniques, and microbiological techniques well known in the art are optionally used. These methods are described in, for example, Ausubel F.A., et al., editors (1988), "Current Protocols in Molecular Biology", Wiley, New York, NY; Sambrook J., et al. (1987), "Molecular Cloning: A Laboratory Manual", 2nd Ed. , Cold Spring Harbor Laboratory Press, Cold Spring Harbor, NY; Bessatsu Jikken Igaku, "Idenshi-Donyu & Hatsugen-Kaiseki-Jikkenho" [Experimantal Medicine, Special Issue, "Experimental Methods for Gene Introduction & Expression Analysis", Yodo-sha, 1997; and the like.

In another aspect, the decellularized tissue, graft, and/or medicament of the present invention comprise instructions which provide guidelines for administration of the decellularized tissue, graft and/or medicament. Here, the instructions describe an appropriate method for administrating the decellularized tissue, graft and/or medicament. The instructions are prepared in a format defined by an authority in a country in which the present invention is used (e.g., the Health, Labor and Welfare Ministry in Japan, Food and Drug Administration (FDA) in the USA, etc.). The instructions explicitly describe the approval by the authority. The instructions are a so-called package insert and are usually provided in a paper medium. The instructions are not limited to this. For example, the instructions may be provided in the form of an electronic medium (e.g., a web site on the Internet, an electronic mail, etc.).

The decellularized tissue, tissue graft, and medicament of the present invention can be implanted using a technique well-known in the art (for surgery, see Hyojun-Geka-Kagaku [Standard Surgical Science], 9th ver. (Igakugakuin), Kihonteki-Geka-Shujutsu-Shugi [Basic Surgical Techniques] (pp. 41-66), Shinzo [Heart] (pp. 349-398), Kekkan [Blood Vessel] (pp. 399-428), and the like). The decellularized tissue of the present invention may be used in vascular anastomosis, cardiovascular reconstruction, vascular prosthesis replacement, prosthetic valve replacement, and the like. Therefore, those skilled in the art can apply the decellularized tissue, tissue graft, and medicament of the present invention in accordance with the disclosure of the present specification depending on circumstances where treatment is performed.

In the present invention, the medicament of the present invention may be applied to any part of the body of an organism. Therefore, the tissue graft may be applied to a part of the body from which the medicament was derived or other parts of the body. As shown in the Examples, it was demonstrated that the desired effect (e.g., regeneration, self organization) of the present invention can be achieved for any part of the body to which the medicament of the present invention is applied, no matter whether or not the part is a site from which the tissue was derived. Therefore, the present invention has a considerably advantageous utility such that the present invention can in principle be applied to any implantation operation and regeneration operation. Examples of parts to which the medicament of the present invention is applied, include, but are not limited to, skin, blood vessel, cornea, kidney, heart, liver, umbilical cord, intestine, nerve, lung, placenta, pancreas, brain, peripheral limb, retina, valve, epithelial tissue, connective tissue, muscluar tissue, nervous tissue, and the like.

Hereinafter, the present invention will be described by way of examples. The following examples are provided only for illustrative purposes. Therefore, the scope of the present invention is limited only by the claims.

EXAMPLES

Hereinafter, the present invention will be described in greater detail by way of examples. The present invention is not limited to the examples below. Reagents and the like used in the following examples are commercially available from Sigma (St. Louis, USA), Wako Pure Chemical Industries, Ltd. (Osaka, Japan), and the like, with exceptions. Animal experiments were conducted in accordance with the guidelines established by Osaka University.

(Example 1)

(Materials and Methods)

(Decellularization by PEG)

Porcine carotid arteries were prepared from Hybrid (Labo Products Co. Ltd., Osaka, Japan), and rat aortas were prepared from SD rats (male, 5 weeks old, Nippon Animal Co., Ltd., Tokyo, Japan) under sterile conditions. Animal experiments were conducted in accordance with the guidelines for ethics established by Osaka University.

Freshly collected porcine carotid arteries and rat aortas were placed in PBS (referred to as PBS (-) in this example; Gibco BRL, Life Technologies Inc. Rockville, MD, USA) containing antibiotics (Gibco BRL, Life Technologies Inc. Rockville, MD, USA) to wash out blood components. The blood vessels were then placed in a decellularizing aqueous solution containing polyethylene glycol (1 g/ml, Nacalai Tesque Inc., Kyoto, Japan) (average molecular weight: 1000), and allowed to stand for 0.5 h. Because of high viscosity of the solution, the blood vessels were gently pressed several times with a glass rod at room temperature. The blood vessels were placed in PBS (-) containing antibiotics (100 units of penicillin, 0.1 mg of streptomycin, 0.25 μg/ml amphotericin B; all of which are available from Gibco BRL, Life Technologies Inc. Rockville, MD, USA) on a rotor (Tube rotator TR-118: Iuchi Co. Ltd, Osaka, Japan) at room temperature. The wash solution was changed every 24 hours over 72 hours. After rinsing, the bloodvessels were immersed in PBS (+) (PBS (-) supplemented with 5 mM MgCl$_2$) containing DNaseI (Takara Shuzo Co., Ltd., Shiga, Japan) at 37°C for 1 h. The blood vessels were placed in the above-described PBS (-) containing antibiotics on a rotor at room temperature. The wash solution was changed every 24 hours over 72 hours. After rinsing, the blood vessels were preserved in PBS (-) containing antibiotics at 4°C.

In addition to PEG having an average molecular weight of 1000, PEGs having average molecular weights 2000, 200, and 6000 were used to perform decellularization treatment as described above. For decellularization treatment, PEG having any molecular weight could be used. It seemed that PEGs having average molecular weight of 2000 and 6000 achieved more satisfactory decellularization treatment, though PEG having an average molecular weight of 1000 was more easily handled in decellularization treatment.

Next, decellularized tissue was prepared in accordance with another decellularization procedure (conventional type (1)=first generation, (2)=second generation). The procedure will be described below.

(First generation conventional decellularization procedure)

1) (Washing) Tissue was washed with physiological saline for one hour at 4°C.

2) (First surfactant treatment) The tissue was placed in physiological saline containing 1% SDS (sodium dodecyl sulfate, SIGMA L-4509) and allowed to stand at room temperature for 48 hours.

3) (Washing) The tissue was removed and was placed again in physiological saline and allowed to stand for 24 hours at room temperature.

4) (Second surfactant treatment) The tissue was placed in physiological saline containing 1% NP-40 (SIGMA I-3021) and was allowed to stand at room temperature for 48 hours.

5) (Washing) The tissue was removed and was placed again in physiological saline and allowed to stand for 24 hours at room temperature.

6) (Sterilization) The tissue was placed in 20% isopropanol (SIGMA I-0398) and was aseptically preserved before use or experiment.

(Second generation conventional decellularization procedure)

1) (Washing) Tissue was placed in physiological saline containing a protease inhibitor (PROTEASE INHIBITOR COCKTAIL; SIGMA P2714) and 20 mM EDTA and washed for 24 hours at 37°C.

2) (First surfactant treatment) The tissue was placed in physiological saline containing 1% SDS (sodium dodecyl sulfate, SIGMA L-4509) and was allowed to stand at room temperature for 72 hours.

3) (Washing) The tissue was removed and was placed in physiological saline and allowed to stand for 48 hours or more at 37°C.

4) (Second surfactant treatment) The tissue was placed in physiological saline containing 1% NP-40 (SIGMA I-3021) and was allowed to stand at room temperature for 48 hours or more.

5) (Washing) The tissue was removed and was placed again in physiological saline for 48 hours at 37°C.

6) (Cryochemistry process) A cryochemistry process was performed in accordance with a commonly used method.

7) (Sterilization) The tissue was placed in 0.05% sodium azide and was asepticallypreserved before use or experiment.

The above-described procedure was performed in accordance with conventional methods.

(Triton treatment)

1) (Washing) Tissue was washed with physiological saline for 1 hour at 4°C.

2) (Surfactant treatment) The tissue (e.g., a blood vessel)was immersed in physiological saline containing 1% Triton (registered trademark) X-100 (ICN Biomedicals, Inc., CA, USA) +0.1% ammonium hydroxide (Wako Pure Chemical Industries Ltd., Osaka, Japan), i.e., a decellularization solution. The decellularization solution was changed every 24 hours using a shaker at 4°C.

3) (Washing) Thereafter, the tissue was washed by changing PBS every 24 hours using a shaker at 4°C a total of three times.

Thus, in conventional treatment of tissue using a surfactant, it is known that SDS and Triton are preferable for the purpose of obtaining desirable physical characteristics. When surfactants, such as SDS and Triton, are in the micellar state, they seem not to have a desired characteristic in terms of the strength and the like. Conversely, it was found that when the surfactants are not in the micellar state, these agents can achieve the object of the present invention. Whereas surfactants form micelles which in turn remove substances, such as proteins, lipids, and the like, an amphipathic molecule (e.g., PEG) as used herein is used in the non-micellar state. Therefore, a solution containing a non-micellar amphipathic molecule is used to remove cell components in accordance with a method for extracting cell components. Thus, this example revealed that the non-micellar amphipathic molecule-containing solution performs decellularization treatment more satisfactorily than a solution containing a micellar molecule, such as a surfactant. Accordingly, it is considered that other non-micelle forming amphipathic molecules can be used for the decellularization treatment of the present invention.

The above-described surfactants could be used to remove cells and cell debris from extracellular matrices of porcine aortic valve tissue. The surfactants used herein were used for the purpose of, particularly, solubilizing phospholipids in cell plasma membrane and intracellular organelles. Amphipathic membrane proteins could also be solubilized with the surfactant. Solubilization was accelerated when the temperature was increased up to 37°C, where diffusion of substances from matrices were promoted.

It has been revealed that collagen structure is affected by pH, ionic strength, the polarity of a solvent, anionic surfactants, and the like (Ripamonti A., et al., 1980, Biopolymers 19:965-975; and Xiao W. H. , Knight D. P. , Chapman J. A., 1997, Biochimica et Biophysica Acta. 1134:327-337). It is also known that a change in collagen structure results in an alteration in bioengineering characteristics. In order to avoid these problems, a decellularization procedure was designed in this example so that matrices are not affected. It was demonstrated by histological tests and tensile strength measurements of the collagen structure that matrices were absolutelynot impaired as follows.

(Histological examination)

Paraffin sections (thickness: 3 $\mu$m) of a blood vessel graft were prepared and stained with hematoxylin-eosin to identify extracellular matrices. To identify I/IV collagen which is a component of the basal membrane, immunohistochemical staining was used. Aortas of SD rats (male, 5 weeks old, Nippon Animal Co., Ltd., Tokyo, Japan) were fixed with 4% paraformaldehyde and cryoprotected. Frozen sections (thickness: 5 $\mu$m) were prepared, followed by permeabilization with PBS (-) for 3 h and then blocking with 1% BSA in PBS (-) for 1 h at room temperature. Subsequently, the sections were incubated with primary antibodies (anti-rat collagen antibodies, Cosmo Bio, Tokyo, Japan), and then with secondary antibodies conjugated with FITC (anti-sheep Ig antibodies; Cosmo Bio, Tokyo, Japan). Images were obtained with a Zeiss LSM 510 confocal microscope.

The results are shown in Figures 1 to 4. Figure 1 shows the results of hematoxylin-eosin staining (H&E) after PEG treatment of the present invention, PEG and DNase treatment of the present invention, or without treatment. Figure 2 shows photographs of porcine aortas with SDS treatment (first generation and second generation) or without treatment. Figures 3 and 4 show photographs results of first generation SDS treatment.

(Endothelial cell (EC) labeling)

Rat endothelial cell (EC) preparation was performed as previously described (Fenselan A., Mello R. J., Cancer Res. 1976, 36, 3269-3273). Briefly, 5-week old SD rats (180-200 g) were killed with carbon dioxide asphyxiation, followed by removal of their aortas. The aortas were immersed in PBS (-) containing 3 mg/ml of collagenase at 37°C for 30 min. The resultant suspension was centrifuged at 900xg for 4 min, and cells were then resuspended in 10 ml DMEM + 10% fetal calf serum. The cells were prepared at $4 \times 10^5$ cells/ml and incubated in 5% $CO_2$ at 37°C.

(Primary culture)

Rat endothelial cells (ECs) were maintained with DMEM (Minimal Essential Medium supplemented with Earle's salt (+) L-glutamine, GibcoBRL, Life Technologies Inc. Rockville, MD, USA) containing 10% fetal calf serum (FBS) (Gibco BRL, Life Technologies Inc. Rockville, MD, USA), 1% non-essential amino acid (Gibco BRL, Life Technologies Inc. Rockville, MD, USA) and antibiotics (100 units of penicillin, 0.1 mg of streptomycin, 0.25 µg/ml amphotericin B; Gibco BRL, Life Technologies Inc. Rockville, MD, USA) at 37°C under 5% $CO_2$ in Falcon tissue culture dishes (Falcon 3003, Beckton-Deckinson Co., Ltd., New Jersey, USA). The cells were washed two times with phosphate-buffered saline (PBS) and 12 ml of medium was added. Thereafter, ECs were labeled with 5 µg/ml cell tracker fluorescent dye Dil (Molecular Probes, Inc., Eugene, OR, USA) at 37°C for 30 min, followed by washing with PBS (-) three times. The thus-labeled ECs were immediately subjected to the following assay.

(Dissemination of ECs on decellularized graft)

A decellularized blood vessel graft was bound to the opposite ends of a nylon thread. A cell suspension was poured into the blood vessel using a 10-ml disposable syringe having a 26-gauge needle for introduction of ECs. ECs at a density of $1.0 \times 10^7$ cells/ml were disseminated in the decellularized graft at room temperature while rotating the graft for one hour. The tissue culture was maintained at 37°C in a humidified atmosphere of 95% air and 5% $CO_2$. The tissue was cultured in the above-described DMEM supplemented with 10% FBS and antibiotics.

(P-D relationship)

Measurement of pressure-diameter (P-D) relationship was carried out in accordance with the Hiromichi Sonoda and Keiichi Takamizawa method (Hiromichi Sonoda et al., J. Biomed. Mater. Res., 2001, 55:266-276). One end of an artery segment was cannulated to a fixed stainless steel connector while the other end was cannulated to a sliding connector. The blood vessel segment was gradually inflated with a pressurized intralumenal Krebs-Ringer solution. The diameter of a middle portion of the blood vessel was monitored using a television system (C1000-16; Hamamatsu Photonics) and a width analyzer (HTV-C1170; Hamamatsu Photonics). The interlumenal pressure of the vessel was simultaneously recorded using a pressure transducer (6M92: NEC Sanei, Tokyo, Japan).

(Results)

As shown in **(c)** and **(d)** in Figure 1, plasma components of the native tissue were eliminated with an aqueous solution containing 1 g/ml PEG (average molecular weight: 1000). The fluidity of the cell membrane was remarkably improved by the high-density PEG aqueous solution. As shown in **(c)** and **(d)** in Figure 1, the cell membrane and plasma components except for the DNA component were observed, because precipitation of the DNA component was confirmed. The aggregation of these components can be explained by the accessibility of the PEG aqueous solution to DNA. This facilitates extraction of cell membrane and cytosol. Persistent degradation and dissolution products of DNA components occurring in tissue grafts can be readily removed with DNaseI. This fact is clearly observed in HE stained images as shown in **(e)** and **(d)** in Figure 1. Formation of cavities was observed between elastic plates due to removal of cell components. The cavity formation seems to suppress degeneration of extracellular matrices. In particular, this treatment had a low apparent effect on collagen components.

As observed with a confocal laser scanning microscope (**(a)** and **(b)** in Figures 5), a slight decrease in Type I collagen was confirmed due to the PEG/DNaseI treatment. As shown in **(c)** and **(d)** in Figure 5, residual Type IV collagen, which is a basal membrane component of blood vessels, was observed. In particular, vascular endothelial cells were easily disseminated for the purpose of antithrombogenicity.

Although a decrease in the mechanical characteristic due to removal of cell components was predicted, the extensibility of the artery blood vessel was maintained (Figure **6**). As the intralumenal pressure increased, the artery was inflated in a pressure-dependent manner. In the case of untreated arteries and PEG/DnaseI-treated arteries, the external diameters thereof were significantly increased in the same manner in a low pressure region up to about 300 mmHg. However, in the physiological pressure range, the pressure-dependent increase became smaller. These features of the P-D relationship indicate that the degeneration of extracellular matrices was suppressed.

(Cell survival rate)

A cell survival rate was calculated by counting the number of nuclei in an area of 100 $\mu$m $\times$ 100 $\mu$m using a microscope. Specifically, the number of nuclei in the same sample was counted in the area before and after treatment. The number of nuclei in the sample after treatment was divided by the number of nuclei in the sample before treatment and the result was multiplied by 100 to obtain a cell survival rate (%). The results are shown in Table 1.

(Tissue injury rate)

A visual field was divided into units of 100 $\mu$m $\times$ 100 $\mu$m and the number of units having an elastin rupture site was counted. There were 24 units per visual field. Elastin rupture was represented by x/24. x was counted when rupture was found in a unit. For example, when no rupture was confirmed (e.g., untreated control), the damage rate was calculated as 0/24, i.e., 0%. The results are summarized in Table 1.

Table 1

|  | Cell survival rate | Tissue damage rate |
|---|---|---|
| No treatment | 100% | 0% |
| First generation SDS process | 38.8% | 40% |
| Second generation SDS process | 4.7% | 15% |
| PEG/DNaseI treatment | 14.6% | 15% |
| TRITON treatment | 17.8% | 34% |

(Tissue strength)

Tissue strength was represented by a $\beta$ value. A $\beta$ value was calculated by the following formula after the P-D relationship curve was prepared:

$$\mathrm{Ln(P/Ps)} = \beta(D/Ds-1) \qquad (1)$$

where P represents a measured pressure, Ps represents a standard pressure (here, 100 mmHg), D represents a measured diameter, and Ds represents a diameter measured at a pressure of 100 mmHg. A greater $\beta$ value indicates a higher stiffness. The thus-obtained $\beta$ values are shown in Table 2. Decellularized tissue by SDS treatment had a $\beta$ value similar to that of decellularized tissue by Triton (registered trademark) treatment (not shown).

Table 2

| Treatment | Ds | $\beta$ value |
|---|---|---|
| No treatment (porcine aorta) | 4.74 | 22.0 |
| PEG/DNaseI treatment | 3.44 | 25.3 |
| Triton (registered trademark) $\times$ 100 treatment | 5.32 | 17.5 |

(Rupture load)

Next, rupture load was investigated. Rupture load was measured as follows: a portion of the aorta wall of a decellularized porcine aortic valve was cut out into a strip of 5 mm in width and 30 mm in length; and the strip was stretched at a test speed of 5 mm/min by a tension testing machine (TENSILON RTC1150A: A&D) to measure the maximum rupture point load (Shinoka T., Mayer J. E., "Tissue Engineering Heart Valve Leaflets", Circulation, 1997; 96 (suppl II):II-102-II-107; Shum-Tim D., Mayer J. E., "Tissue Engineering of Autologus Aorta Using a New Biodegradable Polymer", Ann. Thorac. Surg., 1999; 68:2298-2304). As a result, whereas biological tissue had 1.6±0.9 kgf, PEG-treated tissue had 1.7±0.6 kgf. Further, surfactant-treated tissue had a value significantly lower than these values. Therefore, also, in terms of a parameter, rupture load, it was demonstrated that the PEG treatment of the present invention does not impair physical characteristic.

(Discussion)

Polyethylene glycol is a typical amphiphilic and highly biocompatible polymer. Polyethylene glycol is known to have various utilities by its characteristic molecular properties. For example, polyethylene glycol solution is used for purification of DNA in biological experiments. In another example, polyethylene glycol is used for clinical grade peptide conjugates (see, Veronese F. M. , Biomaterials, 2001, 22:405-417; Monfardini C., Veronese F. M. , Bioconjugate Chem., 1998, 9:418-450; and the like). Polyethylene glycol is used for cell fusion in production of hybridomas. A high density PEG aqueous solution remarkably fluidizes cell membrane containing plasma components, thereby removing the components from tissue. This result was beyond the present inventors' expectation. It is possible to produce decellularized xenograft and allograft tissues by a method using a surfactant (e.g., Tris (registered trademark), SDS, or the like). However, the use of these surfactants is restricted or prohibited in clinical applications, and therefore, it is preferable that these surfactants are not used. In clinical applications of these grafts, it is considered to be desirable that xenograft and allograft tissues are produced without a toxic surfactant and glutaraldehyde fixation. An advantage of the method of the present invention is such that decellularization can be more efficiently performed without a toxic surfactant.

The P(pressure)-D(diameter) relationship of native arteries exhibits a remarkable increase in distensibility in low-pressure regions and little distensibility in high-pressure regions. This P-D relationship is called "J" curve. The effect of compliance has been long discussed as a cause of graft damage during a prolonged period of implantation of an artificial graft having a small diameter (Abott W. M., Semin. Vasc. Surg., 1997; 10-3-7; Pevec W. C., Darling R. C., L'Italien G. J., Abbott W. M., J. Vasc. Surg., 1992; 16:60-65; Bergan J. J., Veith F. J., Bernhard V. M., Yao J. S. T., Flinn W. R., Gupta S. K., Scher L. A., Samson E. H., Towne J. B., Surgery 1982; 92:921-930; and Bos G. W., Poot A. A., Beugeling T., Van Aken W. G., Feijen J., Arch. Physiol. Biochem., 1998; 106:100-115). As shown in Figure 3, the P-D relationship of a PEG/DNaseI-treated porcine arterial vessel exhibits substantially the same characteristic as that of an untreated porcine arterial vessel. In comparison to Triton-X treatment, there is a pressure-dependent difference in compliance. A stiffness parameter ($\beta$ value) was calculated for each artery. The $\beta$ values of PEG/DNaseI- and Triton-X-treated arteries were 22.0 (PEG/DNaseI) and 17.5 (Triton-X). The $\beta$ value of a native artery was 25.3. These data show that a higher order structure of collagen matrices seems to be maintained in PEG/DNaseI-treated grafts. Interestingly, persistence of Type IV collagen, which is a basal membrane component, was observed as shown in (d) in Figure 2. Efficient recellularization of ECs to lumens is deduced from this fact. These observations indicate that extracellular matrices are not much damaged. Recellularization may be more efficiently performed by an improvement in cytophilicity. In addition, a toxic reagent, such as a toxic surfactant, is not used in each step of the method of the present invention. This is a characteristic feature of the present invention. From this finding, it seems that the elasticity of decellularized tissue after treatment is maintained.

In conclusion, cell membrane and plasma components, which seem to cause antigenicity and calcification, may be removed by this method. Therefore, this method makes it possible to more simply and more efficiently produce biological tissue as a useful material. The decellularized tissue of the present invention is useful as a material for regeneration medicine as well as a material for basic research for extracellular matrices. The results obtained by the present inventors suggest that extracellular matrices are not much damaged. In the method of the present invention, the produced material allows detailed analysis of extracellular matrices. The biological tissue may be particularly appropriate for utilization of a native material as an *in vivo* model of variations of a cell in which the three-dimensional structure of its extracellular matrices is affected.

(Example 2: Comparison of reactions within biological tissue)

(Method)

(Immunological response)

Decellularization-treated porcine aortic valves (aorta wall portions (1×1 cm) of a PEG/DNaseI-treated valve and the above-described first generation (SDS, NP-40) treated valve) were implanted under the skins of the dorsal portions of Lewis rats. After one week, the animals were sacrificed. The degree of inflammatory cellular infiltration was scored for evaluation. In this example, porcine native valves and Free Style valves (glutaraldehyde fixation/AOA treatment, conventionally used biological valves) were used as controls for comparison.

(Calcification)

The specimens were collected two months after subcutaneous implantation, followed by von Kossa staining for evaluation of calcification. Also, Ca concentration within the tissue was measured with an atomic absorption spectrometry. The Ca concentration was measured and quantified as follows. The tissue was placed in concentrated hydrochloric acid (or concentrated acid) and was dissolved while heating. Thereafter, atomic absorption spectrometry was performed. The solution was diluted and sprayed into high temperature plasma. Element-specific absorption wavelengths of spectra generated in combustion were measured (Ca: 393.366 nm).

Aorta wall portions (10×10 mm) of decellularization-treated porcine aortic valves were implanted under the skins of the dorsal portions of SD rats (250 g). After two months, the specimens were collected. A Ca concentration (Ca content per dry weight) within the tissue was measured with an atomic absorption spectrometry (SPS7800: Seiko Instrument Inc.) (see, Ozaki S., "Pathophysiology of Calcification of Bioprosthetic Heart Valves", Acta Biomedical Lovaniensia, 2001).

(Implantation)

A portion of aorta wall tissue treated by the first generation decellularization process was implanted to dog descending aortas.

(Results)

Decellularized tissue and control tissue were implanted to 3-week old rats, followed by monitoring for 60 days. In excision, samples were collected for histological evaluation. The samples were stained by hematoxylin-eosin (H&E) staining for evaluation of the whole structure and were stained by von Kossa staining for evaluation of calcification. The results are shown in Figure **7**.

As a result, it was found that: inflammatory cellular infiltration was observed in a plurality of layers in the porcine native valve; and in the decellularized valve graft and the Free Style valve graft, substantially no cellular infiltration was observed, only a slight inflammatory reaction was observed, and the whole tissue structure was not impaired.

Next, the results of evaluation of calcification are shown in Figure **8**. As can be seen from Figure **8**, in the mineralization quality test of von Kossa-stained samples, calcification was confirmed at a surface contacting surrounding tissue of the decellularized valve, while only partial precipitation was observed within the tissue. On the other hand, significant calcification was confirmed in substantially all layers of the glutaraldehyde fixed/AOA treated control sample.

A Ca concentration within the tissue was measured with an atomic absorption spectrometry. As a result, the Ca concentration of the decellularized valve was about 1/10 of the Ca concentration of the glutaraldehyde fixed/AOA treated control samples. It was demonstrated that there was slight calcification in the tissue. Compared to SDS treatment, PEG treatment resulted in a lower Ca concentration within tissue (a graph at the bottom of Figure **8**).

In the experiment in which a portion of aorta wall tissue of the above-described first generation (SDS, NP-40 treatment) valve was implanted into dog descending aortas, one dog was died of graft rupture two weeks after implantation (**(a)** and **(b)** in Figure **9**). Another dog survived until autopsy (no data). All dogs implanted with a valve treated with PEG/DNaseI according to the present invention survived for more than one month. A similar experiment was conducted and valve samples were excised two weeks after implantation. The results are shown in Figure **10**. Figure **10** shows an outer membrane surface of a dog thorax descending aorta implanted with a portion of the first generation process decellularized porcine aortic valve. A photograph of a sample excised two weeks after implantation shows the aneurismal extension of an aorta wall.

(Example 3: Confirmation of cell replacement)

Comparison of Reactions in Biological Tissue between each Valve

Decellularization-treated porcine forearm arteries (a PEG/DNaseI-treated blood vessel and a SDS/NP-40-treated blood vessel) were implanted into dog femoral aortas. The animals were sacrificed after 10 days. The degree of inflammatory cellular infiltration was compared and studied.

(Results)

It was found that there was only a slight inflammatory reaction either in the PEG/DNaseI-treated blood vessel or the SDS/NP-40-treated blood vessel and the whole tissue structure was not impaired in either vessel. The results are shown in Figure 11. However, in the PEG/DNaseI-treated blood vessel, vascular endothelia were confirmed only 10 days after implantation and muscular cell-like nuclei were confirmed in the blood vessel wall. This indicates that the decellularized blood vessel was replaced with self cells.

The conventional decellularized tissue was not replaced with host-derived cells. Therefore, the decellularized tissue of the present invention has a considerably excellent effect which cannot be achieved by conventional techniques.

(Example 4: Decellularization of pericardia)

Next, pericardia were removed from swine and treated with a surfactant solution or a polyethylene glycol solution as described in Example 1. The cell survival rate and tissue damage rate of the treated pericardia were measured. As a result, decellularized pericardia having a tissue damage rate of less than 30% and a cell survival rate of less than 10% were obtained.

(Example 4: Decellularization of pericardia)

Next, pericardia were removed from swine and treated with a surfactant solution or a polyethylene glycol solution as described in Example 1. The cell survival rate and tissue damage rate of the treated pericardia were measured. As a result, decellularized pericardia having a tissue damage rate of less than 30% and a cell survival rate of less than 10% were obtained.

The pericardia were implanted to Lewis rats as described in Example 2 and were examined for inflammatory cellular infiltration and calcification. Decellularized tissue and control tissue were implanted to 3-week old rats, followed by monitoring for 60 days. After excision of the tissue, samples were collected for histological evaluation. The samples were stained by hematoxylin-eosin (H&E) staining for evaluation of the whole structure (Figure 20).

As a result, substantially no cellular infiltration or calcification were observed in the polyethylene glycol-treated tissue, while significant cellular infiltration and calcification were observed in surfactant-treated tissue (Figure **21**).

Next, this decellularized pericardium was implanted into rat cardiac infarct.

(Cardiac infarct rat model)

Lewis male rats were used in this example. The animals were cared for in compliance with the spirit of animal protection in accordance with "Principles of Laboratory Animal Care" prepared by National Society for Medical Research and "Guide for the Care and Use of Laboratory Animals" (NIH Publication No. 86-23, 1985 revised) prepared by Institute of Laboratory Animal Resource and published by National Intitute of Health.

Acute cardiac infarction was induced as described in Weisman H. F. , Bush D. E., Mannisi J. A., et al. "Cellular mechanism of myocardial infarct expansion", Circulation, 1988;78:186-201. Briefly, rats (300 g, 8 weeks old) were anesthetized with pentobarbital, followed by positive pressure breathing. In order to produce rat cardiac infarction models, the chest was opened via the left 4th intercostal and the left coronary artery was ligated at a distance of 3 mm from the root thereof with 8-0 polypropylene thread.

(Implantation of decellularized pericardium tissue)

Recipient rats were anesthetized. The chest was opened via the left 5th intercostals to expose the heart. The rats were divided into two groups, depending on the substance which was administered into a cardiac infarct region of the rat, i.e. group C (no treatment, n=5) and group S (implanted with decellularized pericardium tissue, n=5). The decellularized pericardium was directly implanted into the infarct site two weeks after ligation of the left anterior descending branch.

(Measurement of function of rat heart)

Heart functions were measured by echocardiography (SONOS 5500, produced by Agilent Technologies) 2 weeks, 4 weeks, and 8 weeks after production of the infarction model. Minor axis images were drawn at positions where the left ventricle had the greatest diameter when viewed from the left, using a 12-MHz transducer. In the B-mode, the end systolic area of the left ventricle was measured. In the M-mode, the end diastolic diameter of the left ventricle (LVDd), the end systolic diameter of the left ventricle (LVDs), and the anterior wall thickness of the left ventricle (LVAWTh) were measured to obtain LVEF and LVFS.

(Histological analysis)

Eight weeks after implantation, the heart was removed. The heart was cut along a minor axis. The heart was immersed in 10% formaldehyde solution, followed by paraffin fixation. Sections were prepared, followed by hematoxylin-eosin staining and Masson trichrome staining. At the same time, the sections were frozen, followed by factor VIII immunological staining (Figure 22).

(Results)

Four weeks after implantation, echocardiography was conducted. As a result, the systolic ejection fraction (not shown) and the left ventricle contraction fraction of group S were significantly improved as compared to the other three groups. These functional improvements were maintained 8 weeks after implantation.

(Histological evaluation)

Group S exhibited a significant increase in the LV wall thickness and a significant reduction in the LV cross section area as compared to group C. According to microscopic inspection, it was found that the newly formed cardiac tissue compensated for the infarct in the LV wall.

Thus, it was demonstrated that the decellularized tissue of the present invention can be well applicable to sites other than a site from which tissue is derived before treatment.

(Example 5: Decellularization of Dura mater)

Next, dura maters were removed from swine and were treated with a surfactant or a polyethylene glycol solution as described in Example 1. The cell survival rate and tissue damage rate of the treated dura mater were measured. As a result, decellularized dura maters having a tissue damage rate of less than 30% and a cell survival rate of less than 10% were obtained.

The dura maters were implanted to Lewis rats as described in Example 2 and were examined for inflammatory cellular infiltration and calcification. As a result, substantially no cell filtration or calcification was observed in the polyethylene glycol-treated tissue, while significant cell filtration and calcification were observed in the surfactant-treated tissue. Further, the rats implanted with the polyethylene glycol-treated tissue survived for a significantly longer period of time than the surfactant-treated rats.

Thus, according to the method of the present invention, any tissue can be treated into decellularized tissue having similar excellent characteristics.

(Example 6)

In recent regeneration medicine, attention is attracted by small-diameter vascular prostheses (Kaushal S., Amiel G. E., Guleserian K. J., Shapira O. M., Perry T. , Sutherland F. W., Rabkin E., Moran A.M., Schoen F. J., Atala A., Soker S., Bischoff J., Mayer J. E. Jr., Nat. Med., 7 (2001) 1035-40). At present, techniques of producing small-diameter vascular prostheses are roughly divided into 1) a technique of adhering recipient's self cells to a framework of a biodegradable polymer; and 2) a technique of decellularizing a biological blood vessel (e.g., porcine artery) and adhering recipient's self cells to the decellularized vessel as a framework. The latter technique has an advantage of supplying a stable framework, though decellularization and cell adhesion are not well established. For the latter technique, various methods have been advocated (Bader A., Schilling T., Teebken O. E., Brandes G., Herden T., Steinhof f G., Haverich A., Eur. J. CardioThorac. Surg., 14 (1998) 279-84; O'Brien M. F., Goldstein S., Walsh S., Black K. S., Elkins R., Clarke D. , Semin. Thorac. Cardiovasc. Surg., 11 (1999) 194-200; Steinhoff G. , Stock U., Karim N., Mertsching H., Timke A., Meliss R. R., Pethig K., Haverich A., Bader A., Circulation, 102 (2000) 50-5). In the field of tissue valve development, there is a demand for development of a tissue valve which does not require glutaraldehyde fixation which is a main cause of calcification. Recent remarkable advances in tissue cell engineering have contributed to development of tissue valves having more excellent durability compared to conventional tissue valves. In the field of small-diameter vascular prostheses, a change in characteristics of donor blood vessels treated with decellularization has not fully clarified.

In this example, the present inventors decellularized blood vessels and implanted the vessels into heterologous blood vessels, and histologically examined the vessels over time.

(Methods)

Porcine forearm arteries were prepared from Hybrid (Labo Products Co. Ltd., Osaka, Japan) under sterile conditions. Animal experiments were conducted in accordance with the guidelines for ethics established by Osaka University.

PEG/DNaseI-treated blood vessels were obtained as follows. Freshly collected porcine forearm arteries were placed in PBS containing antibiotics (Gibco BRL, Life Technologies Inc., Rockville, MD, USA) (this solution is referred to as PBS (-) in this example) to wash out blood components. Next, the blood vessels were placed in a decellularizing aqueous solution containing polyethylene glycol (1 g/ml, average molecular weight: 1000, Nacalai Tesque Inc., Kyoto, Japan) and were allowed to stand for 0.5 hours. Because of high viscosity of the solution, the blood vessels were gently pressed several times with a glass rod at room temperature. The blood vessels were placed in PBS (-) containing antibiotics (100 units of penicillin, 0.1 mg of streptomycin, 0.25 µg/ml amphotericin B; all of which are available from Gibco BRL, Life Technologies Inc. Rockville, MD, USA) on a rotor (Tube rotator TR-118: Iuchi Co. Ltd, Osaka, Japan) at room temperature. The wash solution was changed every 24 hours over 72 hours. After rinsing, the blood vessels were immersed in PBS (+) (PBS (-) supplemented with 5 mMMgCl$_2$) containing DNaseI (Takara Shuzo Co., Ltd., Shiga, Japan) at 37°C for 1 h. The blood vessels were placed in the above-described PBS (-) containing antibiotics on a rotor at room temperature. The wash solution was changed every 24 hours over 72 hours. After rinsing, the blood vessels were preserved in PBS (-) containing antibiotics at 4°C. SDS/NP-40-treated blood vessel was placed in physiological saline containing a protease inhibitor (PROTEASE INHIBITOR COCKTAIL; SIGMA P2714) and 20 mM EDTA for washing for 24 hours at 37°C. The tissue was placed in physiological saline containing 1% SDS (sodium dodecyl sulfate, SIGMA L-4509) and was allowed to stand at room temperature for 72 hours. The tissue was removed and was placed in physiological saline and allowed to stand for 48 hours or more at 37°C. The tissue was placed in physiological saline containing 1% NP-40 (SIGMA I-3021) and was allowed to stand at room temperature for 48 hours or more. The tissue was removed and was placed again in physiological saline for 48 hours at 37°C. Finally, the tissue was placed in 0.05% sodium azide and was aseptically preserved before use or experiment.

Implantation was performed as follows. Beagle dogs (5 kg) were generally anesthetized and the above-described decellularization-treated forearm arteries (inner diameter: 3-4 mm, graft length: 5 cm) (PEG/DNaseI-treated blood vessels and SDS/NP-40-treated blood vessels) were implanted into dog femoral aorta portions. The beagle dogs were sacrificed on postoperative day 2, 5, 10 (or 11), and 20, and were histologically examined. Hematoxylin-eosin staining was performed and the number of cells per visual field was counted. Further, elastin, collagen, vascular endothelial cells, and smooth muscle cells were immunologically stained and compared.

(Results)

In the PEG/DNaseI-treated blood vessels, the presence of nuclei within the graft was confirmed from postoperative day 5 according to hematoxylin-eosin staining. On postoperative day 10, the graft had the same number of cells as that in a normal blood vessel (Figures **12A** and **13B**). Victoria blue staining was performed so as to confirm elastin. The presence of elastin was also confirmed in the decellularized blood vessels (Figures **12C, 16**, and **17**). According to sirius red staining (immunological staining) which stains collagen, it was confirmed that elastin was filled with collagen over time (Figures **12C** and **16**). According to factor VIII staining, vascular endothelial cells were confirmed from postoperative day 2 and a layer of endothelial cells was confirmed up until postoperative day 20 (Figure **18**). α-actin positive cells, which were not confirmed on postoperative day 10, were partly confirmed on postoperative day 20 (Figure **19**).

(Discussion)

The development of decellularized vascular prostheses having long-term durability plays an important role in providing novel vascular prostheses for overcoming the drawbacks of conventional vascular prostheses in the field of cardiac surgery. Decellularized vascular prostheses prepared by PEG/DNaseI treatment according to the present invention have the following characteristic features: the prosthesis is obtained by causing self tissue, particularly endothelial cells, to be implanted in a basic vascular framework (or scaffold); inflammatory reactions or immune reactions are reduced; excellent antithrombogenicity and anticalcification characteristics; and long-term durability. Decellularized vascular prostheses have an advantage of supplying stable frameworks. Decellularized porcine arteries are traditionally frequently used as tissue valve materials. However, decellularization techniques are still in the developmental stage. A change in the characteristic of donor arteries due to decellularization has not fully clarified. Various researches have revealed that the durability of biological valves is significantly dependent on calcification but the details have not clarified (Cohn L. H., Collins J. J. Jr., DiSesa V. J., Couper G. S., Peigh P. S., Kowalker W., Allred E., Ann. Surg., 210 (1989) 435-42; Khan S. S., Chaux A., Blanche C., Kass R. M., Cheng W., Fontana G. P., Trento A., Ann. Thorac. Surg., 66 (1998) S35-9; Walley V. M., Keon C. A., Khalili M., Moher D., Campagna M., Keon W. J., Ionescu-Shiley, Ann, Thorac. Surg,, 54 (1992) 111-6; Walley V. M., Keon C. A., Khalili M., Moher D., Campagna M., Keon W. J., Ionescu-Shiley, Ann. Thorac. Surg., 54 (1992) 117-22). In the field of the small-diameter blood vessel, an attempt has been made to disseminate endothelial cells to decellularized blood vessels and ureters of animals, expecting a satisfactory cellular affinity. However, decellularization, in which only the affinity of disseminated cells is taken into account, cannot prevent inflammatory reaction after implantation and the fragility of elastic fibers (Courtman D. R., J. Biomed. Mater. Res., 55 (2001):576-86).

On the other hand, it is unclear as to when self cells are implanted into a scaffold and formed into tissue in the course of self organization of biocompatible blood vessels implanted in organisms.

Niklason et al. reported that by utilizing cell and tissue engineering techniques, a scaffold made of a bioabsorbable polyglycolic acid was used; smooth muscle cells were disseminated; the load of a pulsatile flow was applied; a blood vessel wall having a sufficient strength was produced in a culture; vascular endothelial cells were disseminated; and, as a result, 3 mm-thick full cultured vascular prostheses were successfully produced. Shin-oka et al. reported that vascular endothelial cell collected from a self vein were disseminated in an absorbable scaffold to produce a vascular prosthesis which can grow and the prosthesis was implanted into a pulmonary artery. However, in either report, cells are disseminated before implantation, so that tissue is permitted to be recognized as self tissue. In the present invention, decellularized tissue having a satisfactory cellular affinity is implanted into an organism without cell dissemination before implantation and the tissue is recognized as self tissue over time. Such a technique cannot be found in conventional reports.

The studies conducted by the present inventors revealed that substantially fully decellularized blood vessels were implanted into organisms; the presence of nuclei within the graft was confirmed from day 5 after implantation; and substantially the same number of cells as that of a normal blood vessel were present on postoperative day 10. Further, it was confirmed that cavities between elastic fibers were filled with collagen over time; and on postoperative day 20, $\alpha$-actin positive cells were partly confirmed. This finding is considered to indicate that self cells are implanted into a scaffold and are formed into tissue in the course of self organization of biocompatible blood vessels implanted in organisms. In the future, it is necessary to study the origin of the implanted cells and find a method for causing cells to be more rapidly guided and implanted into decellularized tissue.

As described above, the present inventors observed implanted decellularized vascular prostheses over time and confirmed the course of formation of self tissue. As a result, it was demonstrated that decellularized blood vessels do not tend to elicit an immune reaction, can resist infection and calcification, and have physical characteristics similar to those of native blood vessels.

Further, it was demonstrated that the heterologous decellularized blood vessel of the present invention can act as a scaffold for reconstruction of blood vessels by host cells *in vivo*.

As described above, the present invention is illustrated by way of preferred embodiments and examples, the present invention. It should be understood that the scope of the present invention is limited only by the claims. All patents, patent applications, and publications cited in this specification are herein incorporated by reference in their entireties to the same extent as if each were specifically herein described.

INDUSTRIAL APPLICABILITY

The present invention has established a decellularization technique for reducing a cell survival rate to a level which does not elicit an immune reaction or calcification while reducing a tissue damage rate to a level which permits clinical applications. When decellularized tissue and graft prepared by this technique is implanted into an organism, the organism can survive without eliciting an immune response or calcification. Further, it was confirmed that host-derived cells infiltrate and replace decellularized tissue and graft of the present invention after implantation. Such an event has never taken place in conventional tissue grafts. This is an unexpected, considerably excellent effect of the present invention. Therefore, such tissue is industrially useful. The invention may be described by way of the following numbered paragraphs:

1. Decellularized tissue, wherein:

   A) a cell survival rate of the tissue is less than a level at which an immune reaction is elicited in an organism; and
   B) the tissue is not damaged to such an extent that hinders the tissue from exhibiting a function which was possessed by the tissue when the tissue was not decellularized.

2. Decellularized tissue according to paragraph 1,
wherein the the cell survival rate of the tissue is 30% or less.

3. Decellularized tissue according to paragraph 1,
wherein the tissue damage rate of the tissue is 30% or less.

4. Decellularized tissue according to paragraph 1,
wherein the tissue has a tissue strength which permits a clinical application.

5. Decellularized tissue according to paragraph 1,
wherein the tissue has a tissue strength which is 80% or more of a tissue strength which was possessed by the tissue when the tissue was not decellularized.

6. Decellularized tissue according to paragraph 1,
wherein the tissue has a tissue strength having a P value which is 80% or more of a P value which was possessed by the tissue when the tissue was not decellularized.

7. Decellularized tissue according to paragraph 1,
wherein the tissue has a tissue strength having a β value of 20 or more.

8. Decellularized tissue according to paragraph 1,
wherein the tissue is lumenal tissue.

9. Decellularized tissue according to paragraph 1,
wherein the tissue is tissue selected from blood vessels, blood vessel-like tissue, cardiac valves, pericardia, dura mater, corneas, and bones.

10. Decellularized tissue according to paragraph 1,
wherein a state of the tissue, in which the tissue is not damaged to such an extent that hinders the tissue from exhibiting a function which was possessed by the tissue when the tissue was not decellularized, includes that an extracellular matrix of the tissue is not substantially degenerated.

11. Decellularized tissue according to paragraph 10,
wherein a survival rate of the extracellular matrix is at least about 50%.

12. Decellularized tissue according to paragraph 1,
wherein the tissue is derived from a mammal.

13. Decellularized tissue according to paragraph 1,
wherein the tissue is derived from a human.

14. Decellularized tissue according to paragraph 1,
wherein the tissue is derived from a swine.

15. A tissue graft, comprising:

A) decellularized tissue, wherein a cell survival rate of the tissue is less than a level at which an immune reaction is elicited in an organism; the tissue is not damaged to such an extent that hinders the tissue from exhibiting a function which was possessed by the tissue when the tissue was not decellularized; and the tissue has a desired structure.

16. A tissue graft according to paragraph 15, wherein the tissue graft comprises a cell.

17. A tissue graft according to paragraph 16, wherein the cell is a recipient-derived cell.

18. A tissue graft according to paragraph 16, wherein the cell is derived from an organism of the same species as the tissue.

19. A tissue graft according to paragraph 15, wherein the tissue graft comprises no cell.

20. A tissue graft according to paragraph 15, wherein the decellularized tissue is tissue of an organ selected from the group consisting of blood vessels, blood vessel-like tissue, cardiac valves, pericardia, dura mater, corneas, and bones.

21. A tissue graft according to paragraph 15, wherein the tissue is derived from a mammal.

22. A tissue graft according to paragraph 15, wherein the tissue is derived from a human.

23. A tissue graft according to paragraph 15, wherein the cell is selected from the group consisting of vascular endothelial cells, smooth muscle cells, fibroblast, blood cells, and precursor cells and somatic stem cells capable of differentiating thereto.

24. A membrane-like tissue graft, comprising:

A) decellularized tissue, wherein a cell survival rate of the tissue is less than a level at which an immune reaction is elicited in an organism; the tissue is not damaged to such an extent that hinders the tissue from exhibiting a function which was possessed by the tissue when the tissue was not decellularized; and the tissue has a desired structure.

25. A membrane-like tissue graft according to paragraph 24, wherein the tissue graft comprises a cell.

26. A membrane-like tissue graft according to paragraph 24, wherein the cell is a recipient- derived cell.

27. A membrane-like tissue graft according to paragraph 25, wherein the cell is derived from an organism of the same species as the tissue.

28. A membrane-like tissue graft according to paragraph 25, wherein the tissue graft comprises no cell.

30. A method of producing decellularized tissue, comprising the steps of:

1) providing tissue; and
2) immersing the tissue in a solution containing a non-micellar amphipathic molecule.

31. A method according to paragraph 30, further comprising:

3) washing the tissue.

32. A method according to paragraph 31, wherein the washing step is performed with PBS.

33. A method according to paragraph 30, wherein the amphipathic molecule is a 1,2-epoxide polymer.

34. A method according to paragraph 30, wherein the amphipathic molecule is polyethylene glycol (PEG).

35. A method according to paragraph 34, wherein an average molecular weight of the PEG is between 200 to 6000.

36. A method according to paragraph 34, wherein an average molecular weight of the PEG is between 1000 and 2000.

37. A method according to paragraph 34, wherein an average molecular weight of the PEG is between 1500 and 2000.

38. A method according to paragraph 34, wherein an average molecular weight of the PEG is smaller than or equal to 1000.

39. A method according to paragraph 30, wherein the immersing step is performed for 30 min to 60 min.

40. A method according to paragraph 30, wherein the immersing step comprises physical treatment.

41. A method according to paragraph 30, wherein the washing step is performed for 3 days to 5 days.

42. A method according to paragraph 30, wherein the amphipathic molecule is biocompatible.

43. A method according to paragraph 30, wherein the tissue is tissue selected from the group consisting of blood vessels, blood vessel-like tissue, cardiac valves, pericardia, dura mater, corneas, and bones.

44. A method according to paragraph 30, wherein the tissue is derived from a mammal.

45. A method according to paragraph 30, wherein the tissue is derived from a human.

46. A method according to paragraph 30, further comprising:

4) performing chemical treatment.

47. A method according to paragraph 31, further comprising:

4) performing chemical treatment.

48. A method according to paragraph 46, wherein the chemical treatment is performed with DNase.

49. A method according to paragraph 46, wherein the chemical treatment is performed with DNaseI.

50. A method according to paragraph 47, wherein the chemical treatment is performed with DNase.

51. A method according to paragraph 47, wherein the chemical treatment is performed with DNaseI.

52. A method according to paragraph 30, further comprising:

disseminating a cell.

53. A decellularized tissue, obtained by a method according to paragraph 30.

54. A tissue regeneration method, comprising the steps of:

a) providing decellularised tissue into an organism, wherein a cell survival rate of the tissue is less than a level at which an immune reaction is elicited in an organism; and the tissue is not damaged to such an extent that hinders the tissue from exhibiting a function which was possessed by the tissue when the tissue was not decellularized; and
b) incubating the tissue within the organism for a time sufficient for the tissue to regenerate.

55. A method according to paragraph 54, further comprising providing a cell to the decellularized tissue.

56. A method according to paragraph 55, wherein the cell is derived from the organism.

57. A method according to paragraph 55, wherein the cell is present within the organism.

58. A method according to paragraph 55, wherein the cell is derived from a host homologous to the organism.

59. A method according to paragraph 55, wherein the cell is derived from a host heterologous to the organism.

60. A method according to paragraph 55, wherein the cell is previously isolated from the organism.

61. A method according to paragraph 55, wherein the cell is a blood vessel cell or a blood vessel-like cell.

62. A method according to paragraph 54, further comprising providing a physiologically active substance capable of inducing cell differentiation to the organism.

63. A method according to paragraph 62, wherein the physiologically active substance is derived from or foreign to the organism.

64. A method according to paragraph 62, wherein the physiologically active substance is provided in a form of a nucleic acid or a polypeptide.

65. A method according to paragraph 62, wherein the physiologically active substance is selected from the group consisting of HGF, VEGF, FGF, IGF, PDGF, and EGF.

66. A method according to paragraph 54, wherein the tissue is tissue selected from the group consisting of blood vessels, blood vessel-like tissue, cardiac valves, pericardia, dura mater, corneas, and bones.

67. A method of producing a tissue graft, comprising the steps of:

a) providing decellularized tissue into an organism, wherein a cell survival rate of the tissue is less than a level at which an immune reaction is elicited in an organism; the tissue is not damaged to such an extent that hinders the tissue from exhibiting a function which was possessed by the tissue when the tissue was not decellularized; and

b) causing a self cell in the organism to infiltrate the decellularized tissue; and

c) incubating the tissue within the organism for a time sufficient for the cell to differentiate.

68. A method according to paragraph 52, wherein the cell is a blood vessel cell or a blood vessel-like cell.

69. A method according to paragraph 67, wherein the tissue is tissue selected from the group consisting of blood vessels, blood vessel-like tissue, cardiac valves, pericardia, dura mater, corneas, and bones.

70. A method according to paragraph 67, wherein the decellularization tissue comprises a cell.

71. A method according to paragraph 67, wherein the decellularization tissue is autologous.

72. A method according to paragraph 67, wherein the decellularization tissue is derived from a homologous host.

73. A method according to paragraph 67, wherein the decellularization tissue is derived from a heterologous host.

74. A method according to paragraph 70, wherein the cell is autologous.

75. A method according to paragraph 70, wherein the cell is derived from a homologous host.

76. A method according to paragraph 70, wherein the cell is derived from a heterologous host.

77. A method according to paragraph 67, further comprising:

d) providing a physiologically active substance capable of inducing differentiation of the cell.

78. A method according to paragraph 77, wherein the physiologically active substance is a cytokine having hematopoiesis activity.

79. A tissue graft, produced by a method according to paragraph 67.

80. A method of treating a subject requiring implantation of tissue or an organ or treating a subject at risk of implantation of tissue or an organ for prophylaxis, the method comprising the steps of:

1) providing decellularized tissue, wherein a cell survival rate of the tissue is less than a level at which an immune reaction is elicited in an organism; the tissue is not damaged to such an extent that it hinders the tissue from exhibiting a function which was possessed by the tissue when the tissue was not decellularized; or a tissue graft comprising the decellularized tissue; and

2) implanting the decellularized tissue or tissue graft to the subject.

81. A method according to paragraph 80, wherein the tissue further comprises a cell.

82. A method according to paragraph 81, wherein the cell is a recipient-derived cell.

83. A method according to paragraph 81, wherein the cell is derived from an organism of the same species as the tissue.

84. A method according to paragraph 81, wherein the tissue comprises no cell.

85. A method according to paragraph 80, wherein the tissue is derived from the subject.

86. A method according to paragraph 80, wherein the tissue is tissue selected from the group consisting of blood vessels, blood vessel-like tissue, cardiac valves, pericardia, dura mater, corneas, and bones.

87. A method according to paragraph 80, wherein the subject is a mammal.

88. A method according to paragraph 80, wherein the subject is a human.

89. A medicament for organ implantation, comprising:

A) decellularized tissue, wherein a cell survival rate of the tissue is less than a level at which an immune reaction is elicited in an organism; the tissue is not damaged to such an extent that hinders the tissue from exhibiting a function which was possessed by the tissue when the tissue was not decellularized, or a tissue graft comprising the decellularized tissue.

90. A medicament according to paragraph 89, wherein the tissue is tissue selected from the group consisting of blood vessels, blood vessel-like tissue, cardiac valves, pericardia, dura mater, corneas, and bones.

91. A medicament according to paragraph 89, wherein the cell is derived from a mammal.

92. A medicament according to paragraph 89, wherein the tissue is derived from a human.

93. A medicament according to paragraph 89, wherein the tissue is derived from the subject requiring implantation.

94. A medicament according to paragraph 89, wherein the tissue is derived from a swine.

95. Use of decellularized tissue, wherein a cell survival rate of the tissue is less than a level at which an immune reaction is elicited in an organism; the tissue is not damaged to such an extent that hinders the tissue from exhibiting a function which was possessed by the tissue when the tissue was not decellularized, or a tissue graft comprising the decellularized tissue, for producing a medicament for organ implantation.

**Claims**

1. Decellularized tissue, wherein:

A) a cell survival rate of the tissue is less than a level at which an immune reaction is elicited in an organism;
B) the tissue is not damaged to such an extent that hinders the tissue from exhibiting a function which was possessed by the tissue when the tissue was not decellularized; and
C) the tissue is capable of being infiltrated by host-derived cells after implantation.

2. Decellularized tissue according to claim 1, wherein the the cell survival rate of the tissue is 30% or less.

3. Decellularized tissue according to claim 1, wherein the tissue damage rate of the tissue is 30% or less.

4. Decellularized tissue according to claim 1, wherein the tissue has a tissue strength which permits a clinical application.

5. Decellularized tissue according to claim 1, wherein the tissue has a tissue strength which is 80% or more of a tissue strength which was possessed by the tissue when the tissue was not decellularized.

6. Decellularized tissue according to claim 1, wherein the tissue has a tissue strength having a $\beta$ value which is 80% or more of a $\beta$ value which was possessed by the tissue when the tissue was not decellularized.

7. Decellularized tissue according to claim 1, wherein the tissue has a tissue strength having a $\beta$ value of 20 or more.

8. Decellularized tissue according to claim 1, wherein the tissue is lumenal tissue.

9. Decellularized tissue according to claim 1, wherein the tissue is tissue selected from blood vessels, blood vessel-like tissue, cardiac valves, pericardia, dura mater, corneas, and bones.

10. Decellularized tissue according to claim 1, wherein a state of the tissue, in which the tissue is not damaged to such an extent that hinders the tissue from exhibiting a function which was possessed by the tissue when the tissue was not decellularized, includes that an extracellular matrix of the tissue is not substantially degenerated.

**11.** Decellularized tissue according to claim 10, wherein a survival rate of the extracellular matrix is at least about 50%.

**12.** Decellularized tissue according to claim 1, wherein the tissue is derived from a mammal.

**13.** Decellularized tissue according to claim 1, wherein the tissue is derived from a human.

**14.** Decellularized tissue according to claim 1, wherein the tissue is derived from a swine.

**15.** A tissue graft, comprising:

A) decellularized tissue, wherein a cell survival rate of the tissue is less than a level at which an immune reaction is elicited in an organism; the tissue is not damaged to such an extent that hinders the tissue from exhibiting a function which was possessed by the tissue when the tissue was not decellularized; the tissue has a desired structure; and the tissue is capable of being infiltrated by host-derived cells after implantation.

**16.** A tissue graft according to claim 15, wherein the tissue graft comprises a cell.

**17.** A tissue graft according to claim 16, wherein the cell is a recipient-derived cell.

**18.** A tissue graft according to claim 16, wherein the cell is derived from an organism of the same species as the tissue.

**19.** A tissue graft according to claim 15, wherein the tissue graft comprises no cell.

**20.** A tissue graft according to claim 15, wherein the decellularized tissue is tissue of an organ selected from the group consisting of blood vessels, blood vessel-like tissue, cardiac valves, pericardia, dura mater, corneas, and bones.

**21.** A tissue graft according to claim 15, wherein the tissue is derived from a mammal.

**22.** A tissue graft according to claim 15, wherein the tissue is derived from a human.

**23.** A tissue graft according to claim 15, wherein the cell is selected from the group consisting of vascular endothelial cells, smooth muscle cells, fibroblast, blood cells, and precursor cells and somatic stem cells capable of differentiating thereto.

**24.** A membrane-like tissue graft, comprising:

A) decellularized tissue, wherein a cell survival rate of the tissue is less than a level at which an immune reaction is elicited in an organism; the tissue is not damaged to such an extent that hinders the tissue from exhibiting a function which was possessed by the tissue when the tissue was not decellularized; the tissue has a desired structure; and the tissue is capable of being infiltrated by host-derived cells after implantation.

**25.** A membrane-like tissue graft according to claim 24, wherein the tissue damage rate of the tissue is 30% or less.

**26.** A membrane-like tissue graft according to claim 24, wherein the tissue graft comprises a cell.

**27.** A membrane-like tissue graft according to claim 26, wherein the cell is a recipient-derived cell.

**28.** A membrane-like tissue graft according to claim 26, wherein the cell is derived from an organism of the same species as the tissue.

**29.** A membrane-like tissue graft according to claim 26, wherein the tissue graft comprises no cell.

**30.** Use of a decellularized tissue wherein a cell survival rate of the tissue is less than a level at which an immune reaction is elicited in an organism; the tissue is not damaged to such an extent that hinders the tissue from exhibiting a function which was possessed by the tissue when the tissue was not decellularized; and the tissue is capable of being infiltrated by host-derived cells after implantation, as a medicament.

**31.** Use according to claim 30, wherein a cell is provided to the decellularized tissue.

**32.** Use according to claim 31, wherein the cell is derived from an organism to which the medicament is provided.

**33.** Use according to claim 31, wherein the cell is present within an organism to which the medicament is provided.

**34.** Use according to claim 31, wherein the cell is derived from a host homologous to an organism to which the medicament is provided.

**35.** Use according to claim 31, wherein the cell is derived from a host heterologous to an organism to which the medicament is provided.

**36.** Use according to claim 31, wherein the cell is previously isolated from an organism to which the medicament is provided.

**37.** Use according to claim 31, wherein the cell is a blood vessel cell or a blood vessel-like cell.

**38.** Use according to claim 30, further comprising providing a physiologically active substance capable of inducing cell differentiation to an organism to which the medicament is provided.

**39.** Use according to claim 38, wherein the physiologically active substance is derived from or foreign to the organism.

**40.** Use according to claim 38, wherein the physiologically active substance is provided in a form of a nucleic acid or a polypeptide.

**41.** Use according to claim 38, wherein the physiologically active substance is selected from the group consisting of HGF, VEGF, FGF, IGF, PDGF, and EGF.

**42.** Use according to claim 30, wherein the tissue is tissue selected from the group consisting of blood vessels, blood vessel-like tissue, cardiac valves, pericardia, dura mater, corneas, and bones.

**43.** A method of producing a tissue graft, comprising the steps of:

a) providing decellularized tissue into an organism, wherein a cell survival rate of the tissue is less than a level at which an immune reaction is elicited in an organism; the tissue is not damaged to such an extent that hinders the tissue from exhibiting a function which was possessed by the tissue when the tissue was not decellularized; and
b) causing a self cell in the organism to infiltrate the decellularized tissue; and
c) incubating the tissue within the organism for a time sufficient for the cell to differentiate.

**44.** A method of producing decellularised tissue, comprising the steps of:

1) providing tissue;
2) immersing the tissue in a solution containing a non-micellar amphipathic molecule; and
3) disseminating a cell, wherein the cell is a blood vessel or a blood vessel-like cell.

**45.** A method according to claim 43, wherein the tissue is tissue selected from the group consisting of blood vessels, blood vessel-like tissue, cardiac valves, pericardia, dura mater, corneas, and bones.

**46.** A method according to claim 43, wherein the decellularization tissue comprises a cell.

**47.** A method according to claim 43, wherein the decellularization tissue is autologous.

**48.** A method according to claim 43, wherein the decellularization tissue is derived from a homologous host.

**49.** A method according to claim 43, wherein the decellularization tissue is derived from a heterologous host.

**50.** A method according to claim 46, wherein the cell is autologous.

**51.** A method according to claim 46, wherein the cell is derived from a homologous host.

**52.** A method according to claim 46, wherein the cell is derived from a heterologous host.

**53.** A method according to claim 43, further comprising:

d) providing a physiologically active substance capable of inducing differentiation of the cell.

**54.** A method according to claim 53, wherein the physiologically active substance is a cytokine having hematopoiesis activity.

**55.** A tissue graft, produced by a method according to claim 43.

**56.** Use of a decellularized tissue, wherein a cell survival rate of the tissue is less than a level at which an immune reaction is elicited in an organism; and the tissue is not damaged to such an extent that it hinders the tissue from exhibiting a function which was possessed by the tissue when the tissue was not decellularized; and the tissue is capable of being infiltrated by host-derived cells after implantation; or a tissue graft comprising the decellularized tissue wherein the tissue is capable of being infiltrated by host-derived cells after implantation in the manufacture of a medicament to treat a subject at a risk of implantation of tissue or an organ for prophylaxis.

**57.** Use according to claim 56, wherein the tissue further comprises a cell.

**58.** Use according to claim 57, wherein the cell is a recipient-derived cell.

**59.** Use according to claim 57, wherein the cell is derived from an organism of the same species as the tissue.

**60.** Use according to claim 57, wherein the tissue comprises no cell.

**61.** Use according to claim 56, wherein the tissue is derived from the subject.

**62.** Use according to claim 56, wherein the tissue is tissue selected from the group consisting of blood vessels, blood vessel-like tissue, cardiac valves, pericardia, dura mater, corneas, and bones.

**63.** Use according to claim 56, wherein the subject is a mammal.

**64.** Use according to claim 56, wherein the subject is a human.

**65.** A medicament for organ implantation, comprising:

A) decellularized tissue, wherein a cell survival rate of the tissue is less than a level at which an immune reaction is elicited in an organism; the tissue is not damaged to such an extent that hinders the tissue from exhibiting a function which was possessed by the tissue when the tissue was not decellularized; wherein the tissue is capable of being infiltrated by host-derived cells after implantation, or a tissue graft comprising the decellularized tissue wherein the tissue is capable of being infiltrated by host-derived cells after implantation.

**66.** A medicament according to claim 65, wherein the tissue is tissue selected from the group consisting of blood vessels, blood vessel-like tissue, cardiac valves, pericardia, dura mater, corneas, and bones.

**67.** A medicament according to claim 65, wherein the cell is derived from a mammal.

**68.** A medicament according to claim 65, wherein the tissue is derived from a human.

**69.** A medicament according to claim 65, wherein the tissue is derived from the subject requiring implantation.

**70.** A medicament according to claim 65, wherein the tissue is derived from a swine.

**71.** Use of decellularized tissue, wherein a cell survival rate of the tissue is less than a level at which an immune reaction is elicited in an organism; the tissue is not damaged to such an extent that hinders the tissue from exhibiting a function which was possessed by the tissue when the tissue was not decellularized; and the tissue is capable of being infiltrated by host-derived cells after implantation, or a tissue graft comprising the decellularized tissue wherein the tissue is capable of being infiltrated by host-derived cells after implantation, for producing a medicament for organ implantation.

FIG.1

(a) (b) (c) (d)

(e) (f) (g) (h)

# FIG.2

(a)

(b)

**FIG.3**

(a)

(b)

# FIG.4

(a)

(b)

# FIG.5

(a)

(b)

(c)

(d)

(e)

(f)

**FIG.6**

(a)

(b)

(c)

## FIG.7

**(1 w after subcutaneous implantation to rat)**

| Decellularized value | Porcine native value | Free style |
|---|---|---|

| (HE x200) | (HE x200) | (HE x200) |

| Subcutaneous implantation | Decellularized value | Porcine native value | Free style |
|---|---|---|---|
| Cell infiltration score | 0~1 | 2 | 0~1 |

0: no infiltration, 1: 1-2 cell layers, 2: multiple layers

EP 1 649 879 A2

**FIG.8**

Decellularized valve
(von Kossa x40)

Free Style valve
(von Kossa x40)

Atomic absorption spectrometry measurement

Ca concentration within tissue (mg/g dry weight)

60
50
40
30
20
10
0

DCPV     Free Style

EP 1 649 879 A2

# FIG.9

(a)

(b)

FIG.10

# FIG.11

(a)

Day 10 (SDS)

(b)

Day 10 (PEG)

FIG.12A porcine (Hematoxylin-Eosin) — untreated / PEG+DNase I

# FIG.12B

collagen

untreated

PEG+DNase     I

untreated

PEG+DNase     I

FIG.12C Elastin (Victoria blue)

PEG+DNase I

untreated

## FIG.13A Experimental protocol

1. Collection of porcine artery (inner diameter: 3-4 mm)
2. Decellularization (PEG+DNaseI)
3. Implantation into canine (carotid or femoral a.)
4. Excision of graft

**FIG.13B**        (Hematoxylin-Eosin)

Postoperative day 2        Postoperative day 5

Postoperative day 11        Postoperative day 20

EP 1 649 879 A2

**FIG.14**

Number of cells in graft

EP 1 649 879 A2

FIG.15

(PCNA)

Postoperative day 2

Postoperative day 5

Postoperative day 11

Postoperative day 20

**FIG.16**

(Sirius red, Victoria blue)

Postoperative day 11

EP 1 649 879 A2

FIG.17 (Victoria blue)

Postoperative day 2

Postoperative day 5

Postoperative day 11

Postoperative day 20

# FIG.18 (Factor VIII)

Postoperative day 2       Postoperative day 5

Postoperative day 20

FIG.19

(α SM actin)

Postoperative day 11

Postoperative day 20

**FIG.20**

EP 1 649 879 A2

pericardium PEG                    pericardium PEG

Pericardium decellularized by PEG treatment

**FIG.21**

Pericardium decellularized
by PEG treatment

Pericardium decellularized
by surfactant treatment

**FIG.22**

Implantation of pericardium decellularized by PEG treatment into cardiac infarct, and cardiac muscle tissue